# EUROPEAN PATENT SPECIFICATION

(11) **EP 2 816 962 B1**
(45) Date of publication and mention of the grant of the patent: **30.11.2016**
(21) Application number: 13710642.3
(22) Date of filing: 20.02.2013
(51) Int. Cl.: A61B 17/16, A61B 17/17, A61F 2/34, A61F 2/46

(54) **PATIENT-SPECIFIC ACETABULAR GUIDES AND ASSOCIATED INSTRUMENTS**
PATENTSPEZIFISCHE HÜFTGELENKPFANNENFÜHRUNG UND ZUGEHÖRIGE INSTRUMENTE
GUIDES ACÉTABULAIRES ATTRIBUÉS À UN PATIENT ET INSTRUMENTS ASSOCIÉS

(30) Priority: 21.02.2012 US 201213400652
(43) Date of publication of application: 31.12.2014
(73) Proprietor: Biomet Manufacturing, LLC, Warsaw, IN 46582 (US)
(72) Inventor: MERIDEW, Jason D., Warsaw, IN 46580 (US); WITT, Tyler D., Warsaw, IN 46582 (US); SCHOENEFELD, Ryan J., Fort Wayne, IN 46804 (US); SLONE, W. Jason, Silver Lake, IN 46982 (US); METZGER, Robert, Wakarusa, IN 46573 (US); LOMBARDI, Adolph V., New Albany, OH 46054 (US); NYCZ, Jeffrey H., Warsaw, IN 46582 (US); MUNSTERMAN, Matthew D., Warsaw, IN 46582 (US); WOLFE, Alexander P., Columbia City, IN 46725 (US); KABATA, Tamon, Ishikawa 920-8641 (JP); NAIRUS, James G., Concord, MA 01742 (US); CLOHISY, John C., St. Louis, MO 63124 (US)
(74) Representative: Mays, Julie
(86) International application number: PCT/US2013/026875
(87) International publication number: WO 2013/126416

(56) References cited:
- EP-A2- 1 634 551
- WO-A1-2010/124164
- US-A1- 2010 016 984
- US-A1- 2010 274 253
- US-A1- 2011 184 419

## Description

### INTRODUCTION

The present teachings provide a patient-specific acetabular guide and related instruments for preparing an acetabulum to receive an acetabular implant and guiding the implant into the acetabulum of a patient.

### SUMMARY

Document US2011184419 A1 discloses an acetabular guide combination according to the preamble of claim 1. The present teachings provide various instruments for generally preparing the acetabulum of a patient to receive an acetabular implant, such as, for example, an acetabular cup along an alignment axis. The alignment axis and various patient-specific guides and instruments can be designed during a pre-operative plan using a three-dimensional reconstruction of the patient's relevant anatomy, such as the pelvis or portions thereof, including the acetabular and periacetabular areas of the pelvis. The three-dimensional reconstruction can be based on two-dimensional medical images, including MRI, CT or X-ray scans and prepared using commercially available imaging software.

The present teachings provide, for example, an acetabular guide that includes a patient-specific engagement surface designed to be complementary and mateable with a corresponding surface of the patient's pelvic anatomy. The acetabular guide is designed during the pre-operative plan for the patient using the three-dimensional reconstruction of the anatomy of the patient. The patient-specific engagement surface has a first portion mateable with a portion of the acetabulum of the patient. The acetabular guide includes a guiding element that extends from the acetabular guide opposite to the first portion of engagement surface. The guiding element defines a bore designed to be oriented along an alignment axis for an acetabular implant when the acetabular guide is engaged to the acetabulum. A drilling element with a stop can be used to drill a pilot hole into the acetabulum along the alignment axis.

The acetabular guide can be provided in various fitment options in which the patient-specific engagement surface is designed to fit in a unique position relative to the patient anatomy. Each fitment option of the acetabular guide includes a portion that covers a center of the acetabulum for aligning the acetabular implant and additional portions complementary to a portion of the acetabular rim and/or a portion of the transverse acetabular ligament. Each fitment option allows the acetabular guide to have a compact size, extend through the center of the acetabulum for alignment, and include portions that can fit over various anatomic landmarks in a unique position for the patient. The particular fitment option can be selected for each specific patient based on the patient's anatomy, the procedure to be performed and the surgeon's preference and/or technique.

The acetabular guide includes first and second marker elements extending from a portion of the acetabular guide outside the acetabulum of the patient. The marker elements define corresponding first and second bores for guiding first and second marker pins into a bone portion of the patient. A secondary guide is used with the marker pins to orient an acetabular cup into a predetermined position and orientation. The secondary guide is designed during the pre-operative plan to include first and second guiding elements complementary to the first and second marker elements of the acetabular guide for receiving the first and second marker pins when the first and second marker pins are attached to the bone portion of the patient.

The acetabular guide can be used according to the present teachings with a guiding handle support device that can be attached to the pelvis and provides an alignment rod as a reference for the alignment axis. The guiding handle can be removably attached to the guiding element of the acetabular guide along the alignment axis. The support device can include a connector supporting the alignment rod. The connector can be removably engaged with a shaft of the guiding handle. The support device includes rotational and translational mechanisms for orienting the alignment rod parallel to the shaft of the guiding handle and parallel to the alignment axis such that the alignment rod can provide a reference axis for the alignment axis.

The present teachings also provide a reamer having a guiding pin. The guiding pin of the reamer can be received in the pilot hole that is drilled in the acetabulum through the bore of the guiding element of the acetabular guide. The guiding pin is thus oriented along the alignment axis. In some embodiments, the reamer includes a spring that biases the guiding pin and provides tactile feedback during reaming. In some embodiments, the reamer includes a plurality of removable arcuate blades. The blades can be disposable or replaceable. In some embodiments, each blade is attached to a corresponding supporting element shaped as a spherical section, such that the supporting elements collectively form a surface corresponding to a shape of the acetabular cup.

The various instruments described above can be used in various combinations reaming the acetabulum and inserting an acetabular implant according to a pre-operative plan. Also described herein are methods for reaming and preparing an acetabulum of a patient for an acetabular implant. One method includes engaging an acetabular area of the patient with a complementary surface of a patient-specific acetabular guide, supporting an alignment rod on a support device attached to the patient's pelvis and orienting the alignment rod to be parallel to an alignment axis for inserting the implant. The alignment axis is determined during a preoperative plan of the patient and coinciding with a center axis of a guiding element of the acetabular guide. The method further includes drilling a pilot hole in the acetabulum through a bore of the guiding element along the center axis, removing the acetabular guide, guiding an alignment pin of a reamer in the pilot hole such that the alignment pin is parallel to the alignment rod, and reaming the acetabulum.

Another method includes engaging an acetabular area of the patient with a complementary surface of a patient-specific acetabular guide. The acetabular guide is designed during a pre-operative plan from a reconstructed three-dimensional image of the patient's anatomy. The method includes drilling a pilot hole in the acetabulum through a bore of a guiding element of the acetabular guide along a patient-specific alignment axis determined by the guiding bore. The method also includes inserting first and second marker pins into an area outside the acetabulum of the patient through corresponding first and second marker elements of the acetabular guide. The method includes removing the acetabular guide without removing the marker pins, reaming the acetabulum, inserting an acetabular cup in the reamed acetabulum and sliding a secondary guide over the first and second marker pins. The method further includes engaging a planar arcuate surface of the secondary guide to a complementary rim surface of the acetabular cup to align the acetabular cup to a position and orientation determined in the pre-operative plan.

Another method includes using an electronic positioner for guiding an acetabular inserter into the acetabulum along a predetermined patient-specific alignment axis. The electronic positioner is capable of indicating a predetermined orientation using one or more orientation sensors. A patient-specific acetabular guide is placed on the acetabulum of the patient in a unique mated position. The acetabular guide has a guiding element oriented along the patient-specific alignment axis. A shaft of the acetabular inserter is inserted into the guiding element of the acetabular guide, such that the shaft of the acetabular inserter is oriented along the alignment axis. The electronic positioner is removably attached to the shaft of the acetabular inserter and is calibrated to indicate the alignment axis. After the acetabular guide is removed and the acetabulum prepared, the acetabular inserter with the acetabular implant can be guided along the alignment axis using feedback from the electronic positioner.

Further areas of applicability of the present teachings will become apparent from the description provided hereinafter. It should be understood that the description and specific examples are intended for purposes of illustration only and are not intended to limit the scope of the present teachings.

Only the acetabular guide 200 (200A, 200B, 200C) and the secondary guides 600A and 600B fall wihin the scope of the claims. The acetabular guide 100 (100A, 100B, 1000C) and the secondary guide 600 do not fall within the scope of the claims. 30

### BRIEF DESCRIPTION OF THE DRAWINGS

The present teachings will become more fully understood from the detailed description and the accompanying drawings, wherein:
FIGS. 1-5 illustrate environmental perspective views of various patient-specific acetabular alignment guides according to the present teachings;
FIG. 6 is an environmental perspective view of various instruments illustrating a method for establishing an acetabular cup insertion axis according to the present teachings;
FIG. 7 is an environmental perspective view illustrating drilling a pilot hole for guided reaming according to the present teachings;
FIG. 8A is an environmental perspective view illustrating a reamer for guided reaming according to the present teachings;
FIG. 8B is a stylized perspective view of a reamer for guided reaming according to the present teachings;
FIG. 8C is a partially sectioned perspective view of the reamer of FIG. 8B;
FIG. 9 is an environmental perspective view illustrating instruments for cup insertion according to the present teachings;
FIG. 10 is an environmental perspective view of a patient-specific acetabular alignment guide with alignment pins for a secondary guide according to the present teachings;
FIG. 11 is an environmental perspective view of another patient-specific acetabular alignment guide with alignment pins for a secondary guide according to the present teachings;
FIG. 12 is an environmental perspective view of the patient-specific acetabular alignment guide of FIG. 10 illustrating drilling a pilot hole for guided reaming according to the present teachings;
FIG. 13 is an environmental perspective view of a secondary guide over the alignment pins of FIG. 10 according to the present teachings;
FIG. 14 is an environmental perspective view of a patient-specific acetabular alignment guide with an alignment pin and supporting instruments according to the present teachings;
FIG. 15 is an environmental view of another embodiment of a patient-specific acetabular alignment guide shown with marker pins according to the present teachings;
FIG. 16 is an environmental view of another embodiment of a secondary guide shown with marker pins according to the present teachings;
FIG. 17 is an environmental view of another embodiment of a secondary guide shown with marker pins according to the present teachings;
FIG. 18 is an environmental view illustrating a method of inserting an acetabular cup using the secondary guide of FIG. 16;
FIG. 19 is an environmental view illustrating another method of inserting an acetabular cup using an acetabular inserter coupled to the secondary guide of FIG. 16 with an adaptor according to the present teachings;
FIGS. 20A and 20B are perspective views of a detail of coupling between the acetabular inserter and the adaptor of FIG. 19;
FIG. 21 is a perspective view of another embodiment of an adaptor for coupling an acetabular inserter to a secondary guide according to the present teachings;
FIG. 22 is an environmental perspective view illustrating calibrating an electronic positioner coupled to an acetabular inserter and using a patient-specific alignment guide without marker pins according to the present teachings;
FIGS. 23A and 23B illustrate an acetabulum before and after reaming, respectively;
FIG. 24 is an environmental perspective view illustrating inserting an acetabular cup using the calibrated positioner of FIG. 22 according to the present teachings;
FIG. 25 is an environmental perspective view illustrating calibrating an electronic positioner coupled to an acetabular inserter and using a patient-specific alignment guide with marker pins according to the present teachings;
FIGS. 26A and 26B illustrate an acetabulum before and after reaming, respectively, using a secondary guide for alignment during reaming according to the present teachings;
FIG. 27 is an environmental perspective view illustrating inserting an acetabular cup using the calibrated positioner and secondary guide of FIG. 25 according to the present teachings;
FIG. 28 is a perspective view of an electronic positioner coupled to an acetabular inserter/impactor as disclosed in commonly assigned U.S. Patent Publication 2010/0249796; and
FIG. 29 is a schematic view of the electronic positioner of FIG. 28.

### DESCRIPTION OF VARIOUS EMBODIMENTS

The following description is merely exemplary in nature and is in no way intended to limit the present teachings, applications, or uses.

The present teachings generally provide various patient-specific acetabular alignment guides, secondary guides, reamers, inserters, impactors and other associated instruments for use in orthopedic surgery, such as in joint replacement or revision surgery, for example. The patient-specific alignment guides and associated instruments can be used either with conventional or with patient-specific implant components prepared with computer-assisted image methods.

As described in commonly assigned U.S. Application No. 11/756,057, filed on May 31, 2007, during a preoperative planning stage, imaging data of the relevant anatomy of a patient can be obtained at a medical facility or doctor's office. The imaging data can include, for example, a detailed scan of a pelvis, hip, knee, ankle or other joint or relevant portion of the patient's anatomy. The imaging data can be obtained using an MRI, CT, and X-Ray, ultrasound or any other imaging system. The imaging data obtained can be used to construct a three-dimensional computer image of the joint or other portion of the anatomy of the patient and prepare an initial pre-operative plan that can include bone or joint preparation, including planning for resections, milling, reaming, broaching, implant selection and fitting, design of patient-specific guides, templates, tools and alignment protocols for the surgical procedure.

Computer modeling for obtaining three-dimensional computer images of the relevant patient's anatomy can be provided by various CAD programs and/or software available from various vendors or developers, such as, for example, from Materialise USA, Plymouth, Michigan. The computer modeling program can be configured and used to plan a preoperative surgical plan, including planning various bone preparation procedures, to select or design/modify implants and design patient-specific guides and tools including patient-specific prosthesis components, and patient-specific tools, including reaming, broaching, milling, drilling or cutting tools, alignment guides, templates and other patient-specific instruments.

The pre-operative plan can be stored in any computer storage medium, in a computer file form or any other computer or digital representation. The pre-operative plan, in a digital form associated with interactive software, can be made available via a hard medium, a web-based or mobile or cloud service, or a cellular portable device to the surgeon or other medical practitioner, for review. Using the interactive software, the surgeon can review the plan, and manipulate the position of images of various implant components relative to an image of the anatomy. The surgeon can modify the plan and send it to the manufacturer with recommendations or changes. The interactive review process can be repeated until a final, approved plan, is sent to a manufacturing facility for preparing the actual physical components.

After the surgical plan is approved by the surgeon, patient-specific implants and associated tools, including, for example, alignment guides, cutting/milling/reaming/broaching or other tools for the surgical preparation of the joint or other anatomy portion of the specific patient can be designed using a CAD program or other three-dimensional modeling software, such as the software provided by Materialise, for example, according to the preoperative surgical plan. Patient-specific guides and other instruments can be manufactured by various stereolithography methods, selective laser sintering, fused deposition modeling or other rapid prototyping methods. In some embodiments, computer instructions of tool paths for machining the patient-specific guides and/or implants can be generated and stored in a tool path data file. The tool path data can be provided as input to a CNC mill or other automated machining system, and the tools and implants can be machined from polymer, ceramic, metal or other suitable material depending on the use, and sterilized. The sterilized tools and implants can be shipped to the surgeon or medical facility for use during the surgical procedure.

Patient-specific implants, guides, templates, tools or portions thereof are defined herein as those constructed by a surgical plan approved by the surgeon using three-dimensional images of the specific patient's anatomy and made to closely conform and mate substantially as a negative mold or negative surface or inverse or mirror surface of corresponding surface portions of the patient's anatomy, including bone surfaces with or without associated soft tissue, such as articular cartilage, for example, depending on the particular procedure, implant and tool use.

Patient-specific alignment guides and implants are generally configured to match the anatomy of a specific patient and can fit in only one position on a corresponding surface of the specific patient because anatomic features that are unique to each patient function as landmarks and can guide placement of the alignment guide or implant in only one position without the need of intraoperative navigation, patient marking or other intraoperative guidance.. The patient-specific alignment guides are generally configured and manufactured using computer modeling based on the patient's 3-D anatomic image and have an engagement surface that is made to conformingly contact and match as a mirror or negative or inverse surface to a corresponding surface of a three-dimensional image/model of the patient's bone surface (with or without cartilage or other soft tissue), by the computer methods discussed above. The patient-specific alignment guides can include custom-made guiding formations, such as, for example, guiding bores or cannulated guiding posts or cannulated guiding extensions or receptacles that can be used for supporting or guiding other instruments, such as drill guides, reamers, cutters, cutting guides and cutting blocks or for inserting pins or other fasteners according to a surgeon-approved pre-operative plan. The patient-specific alignment guides can be used in minimally invasive surgery, and also in surgery with multiple minimally-invasive incisions. Various alignment guides and pre-operative planning procedures are disclosed in commonly assigned and co-pending U.S. Patent Application No. 11/756057, filed on May 31, 2007, U.S. Patent Application No. 12/211407, filed September 16, 2008; U.S. Patent Application No. 11/971390, filed on January 9, 2008, U.S. Patent Application No. 11/363548, filed on February 27, 2006; and U.S. Patent Application No 12/025414, filed February 4, 2008. The disclosures of the above applications are incorporated herein by reference.

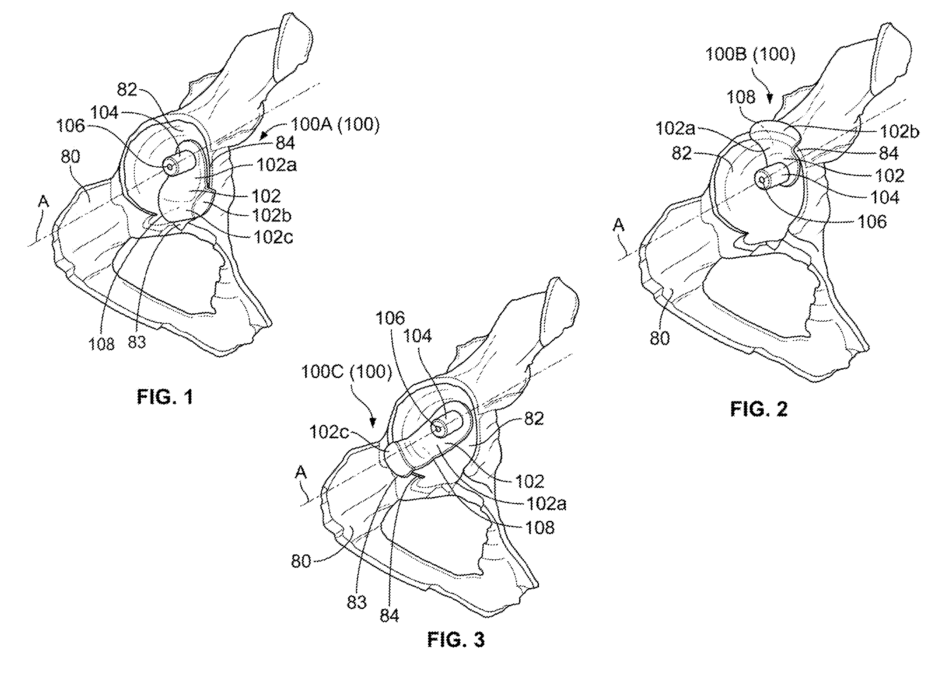

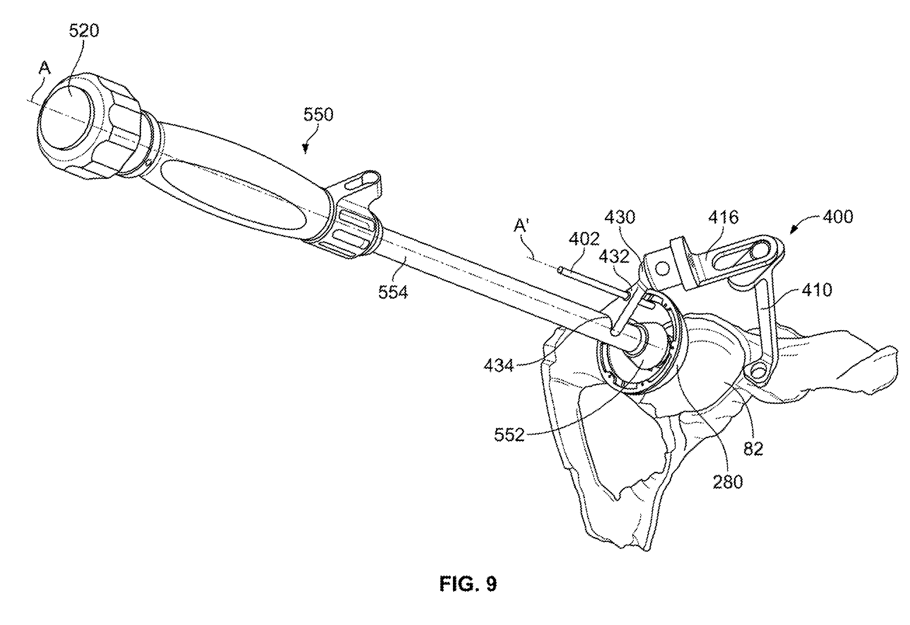

Referring to FIGS. 1-5, the present teachings provide various patient-specific acetabular guides 100, 200. The acetabular guides 100, 200 can be used in connection with various other instruments to facilitate guided reaming of an acetabulum 82 of a pelvis 80 of a specific patient and guided insertion and implantation of an acetabular implant or acetabular cup in the acetabulum 82. Further, the patient-specific acetabular guides 100, 200 engage the acetabulum 82 of the specific patient in a unique (only one) position and can provide an accurate alignment axis relative to the planned orientation of the acetabular cup 280 (shown in FIG. 9, for example). The patient-specific acetabular guides 100, 200 can also provide secure fitting and rotational stability in a design that is lightweight with minimal size and bulk.

FIGS. 1-3 illustrate a patient-specific acetabular guide 100 having a patient-specific body 102, as described below, and a guiding or pilot element 104 having an elongated bore 106 with a patient-specific alignment axis A for drilling a pilot hole on the acetabulum and/or establishing the alignment axis A for a reamer, implant inserter, impactor or other instruments used in the preparation and implantation procedure. The alignment axis A is configured to be central to the acetabular cup and perpendicular to the acetabular cup's surface when the acetabular guide 100 is positioned on the acetabulum 82. The alignment axis A helps orient and guide the acetabular implant and various related instruments along a patient-specific orientation established during the pre-operative plan for the patient. The acetabular guide 100 can be provided in various fitment options depending on the planned exposure of the acetabulum 82 for the reaming procedure and implantation. Each fitment option of the acetabular guide can generally include a portion that covers a center of the acetabulum for aligning the acetabular implant and additional portions complementary to a portion of the acetabular rim and/or a portion of the transverse acetabular ligament. Each fitment option allows the acetabular guide 100 to have a compact size, extend through the center of the acetabulum 82 for alignment, and include portions that can fit over various anatomic landmarks in a unique position for the patient. The particular fitment option can be selected for each specific patient based on the patient's anatomy, the procedure to be performed and the surgeon's preference and/or technique.

Three exemplary fitment options designated 100A, 100B and 100C are illustrated in FIGS. 1-3, respectively. The fitment options can include fitments engaging or registering to various combinations of portions of the acetabulum 82, the acetabular rim 84 and the transverse acetabular ligament 83. For example, the acetabular guide 100 in the fitment option 100A may engage a portion of the acetabulum 82, a portion of the acetabular rim 84 and a portion of the transverse acetabular ligament 83. In the fitment option 100B, the acetabular guide 100 may engage a portion of the acetabulum 82 and a portion of the acetabular rim 84. In the fitment option 100C, the acetabular guide 100 may engage a portion of the acetabulum 82 and a portion of the transverse acetabular ligament 83. Further details are discussed below. Either one or several acetabular guides 100A, 100B, 100C corresponding to different fitment options can be provided to the surgeon for intra-operative flexibility and plan change, according to the surgeon's preference. The acetabular guide 100 can be secured to the patient's bone with bone pins, guide wires or other fasteners.

The patient-specific body 102 of the acetabular guide 100 can include an inner portion 102a (all fitment options) from which the guiding element extends and which is designed to engage the acetabulum 82, an outer portion 102b which extends from the inner portion 102a and is configured to extend over a portion of the rim 84 (for fitment options 100A and 100C) and an outer portion 102c (fitment options 100A and 100C) configured to extend over a portion of the transverse acetabular ligament 83 (and adjacent area of the acetabulum 82). The patient specific body 102 has an underside three-dimensional engagement surface 108 that is custom-made or patient-specific to conform to and mirror (as a negative or inverse or mirror surface) complementary surfaces of various combinations of the acetabulum 82, rim 84 and/or transverse acetabular ligament 83 or other periacetabular surfaces of the pelvis 80 of the specific patient, as described above in connection with the various fitment options. The patient specific body 102 is designed by using a three-dimensional image or model of the acetabulum 82 and surrounding pelvic area of the patient, as described above. The engagement surface 108 enables the acetabular guide 100 to nest or closely mate relative to the complementarily acetabular surface of the patient. The acetabular guide 100 can be designed to have generally small thickness, such that it can form a lightweight three-dimensional shell from which the guiding element 104 extends opposite to the engagement surface. The guiding element 104 can be formed to be a monolithic or integral portion of the acetabular guide 100. Alternatively, the guiding element 104 can be modularly and removably coupled to the acetabular guide 100, using, for example, a threaded connection, snap-on connectors or other removable attachments.

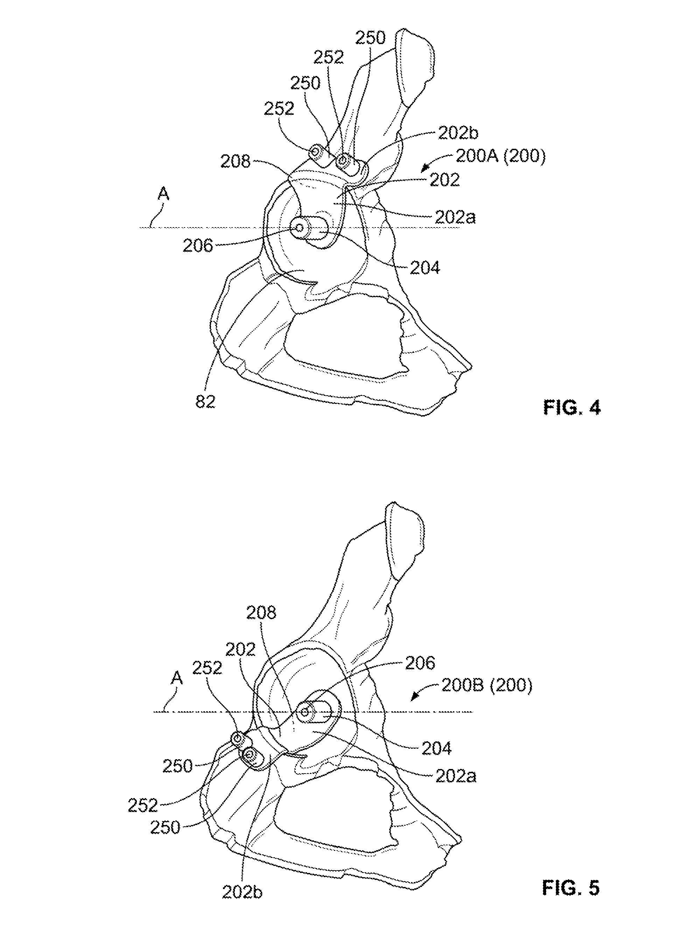

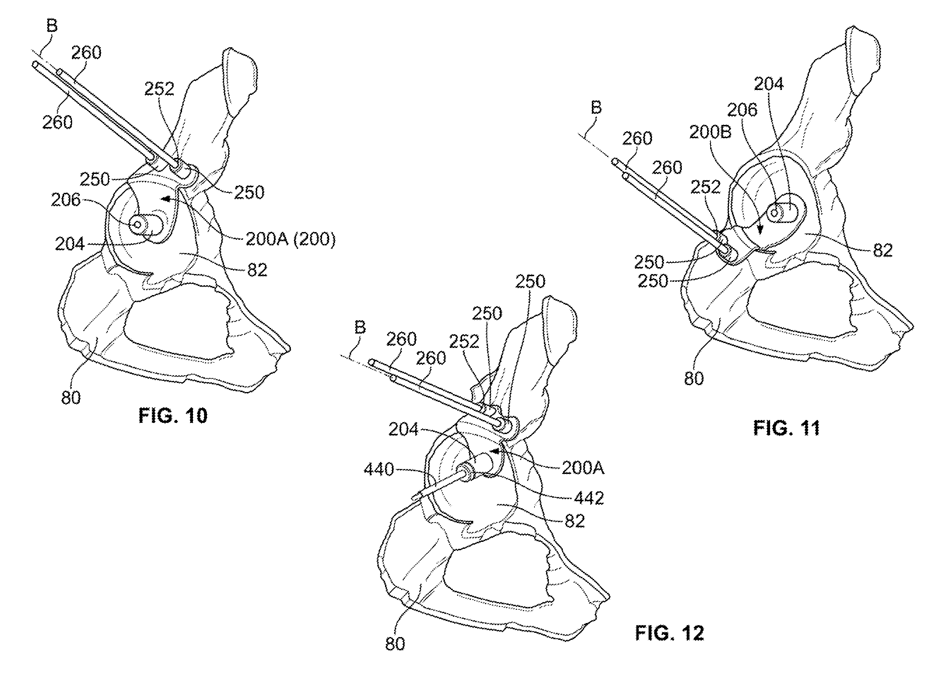

Referring to FIGS. 4 and 5, another patient-specific acetabular guide 200 is illustrated with exemplary two fitment options 200A and 200B. Similarly to the acetabular guide 100, the acetabular guide 200 also includes a patient-specific body 202 and a guiding or pilot element 204 having an elongated bore 206 with an alignment axis A configured to be central to the acetabular cup and perpendicular to the acetabular cup's surface when the acetabular guide 200 is positioned on the acetabulum 82. The acetabular guide 200 can include one or more marker elements 250 (two are shown in the exemplary embodiments of FIGS. 4 and 5), each having an elongated bore 252 for guiding marker pins 260. The marker pins 260 can be used for supporting a secondary guide for another preparation method discussed below in reference to FIG. 12. The other features of the acetabular guide 200 are similar to that of the acetabular guide 100, such that the acetabular guide 200 can also be used instead of the acetabular guide 100. The acetabular guide 100 can be used for procedures in which the marker elements 250 are not utilized, as described below. The acetabular guide 200 can be used for procedures in which the marker elements 250 may or may not be utilized, as described below.

The patient-specific body 202 of the acetabular guide 200 is generally similar to patient-specific body 102 of the acetabular guide 100, such that the patient-specific body 202 can include an inner portion 202a from which the guiding element extends and which is designed to engage the acetabulum 82, and an outer portion 202b which extends from the inner portion 202a and is configured to extend over a rim portion 84 of the acetabulum 82. The outer portion 202b extends sufficiently beyond the rim 84 to the periacetabular area of pelvis to accommodate the marker elements 250. The patient specific body 202 has an underside or bone-engaging three-dimensional engagement surface 208 that is custom-made or patient-specific to conform and mirror (as an inverse or negative or mirror surface) complementary surfaces of selected portions of the acetabulum 82, the rim 84 and the transverse acetabular ligament 83 (depending on the fitment option) or other periacetabular surfaces of the pelvis 80 of the specific patient by using a three-dimensional image or model of the acetabulum and surrounding pelvic area of the patient, as described above. The engagement surface 208 enables the acetabular guide 100 to nest or closely mate relative to the complementarily acetabular surface of the patient. The acetabular guide 200 can be designed to have generally small thickness, such that it can form a lightweight three-dimensional shell from which the guiding element 204 and marker elements 250 extend.

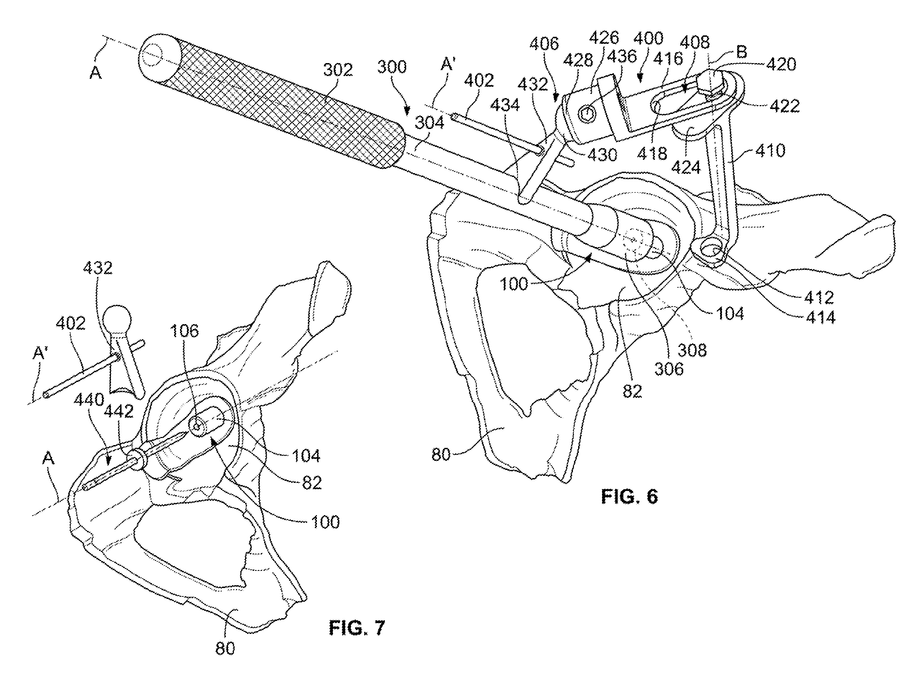

Referring to FIGS. 6-9, a method for reaming and preparing the acetabulum for an implant is described in connection with the patient-specific acetabular guides 100. The acetabular guides 200 can also be used, although the marker elements 250 are not utilized in this method. Referring to FIG. 6, a patient-specific acetabular guide 100 (or 200) is placed in a unique position on the acetabulum/rim/transverse acetabular ligament depending on the fitment option and associated anatomic landmarks of the patient, as determined in the preoperative plan for the specific patient. Once positioned, the guiding element 104 establishes the alignment axis A for the specific patient. An elongated guiding handle 300 can be attached to the guiding element 104 such that the center axis of the guiding handle 300 coincides with the alignment axis A. The guiding handle 300 can include a proximal gripping portion 302, an elongated shaft 304 extending from the gripping portion 302 and a coupling distal portion 306 which can be removably coupled to the guiding element 104, such that the guiding handle 300 is aligned along the alignment axis A. The connection can be a slidable or threadable or other connection between the distal portion 306 and the guiding element 104. The distal portion 306 can include, for example, a bore 308 for receiving the guiding element 104. The guiding element 104 and the bore 308 can be of sufficient length for the guiding handle 300 to be removably yet stably coupled to the guiding element 104 for indicating the alignment axis A without wobbling or other misaligning motion.

With continuing reference to FIG. 6, a support device or jig or outrigger 400 can be secured on the pelvis 80. The support device 400 can be used to orient an alignment pin or rod 402 along an axis A' parallel to the alignment axis A. More specifically, the support device 400 can include a universal rotational adjustment mechanism 406 and a pivotable/translational adjustment mechanism 408 for removably engaging the shaft 304 and aligning the alignment rod 402 parallel to the shaft 304 and, therefore, parallel to the alignment axis. In the exemplary embodiment of FIG. 6, the support device 400 can include a leg 410 that can be attached to the bone with a bone fastener (not shown) through a hole 412 of a foot or base 414 of the leg 410. The support device 400 can also include an arm 416 that is slidable coupled to the leg 410 to allow for translational motion of the arm 416 relative to the leg 410. The arm 416 can have, for example, an elongated slot 418 that slidably receives a fastener head 420 of a fastener 422, such as a screw or bolt that is received through a distal flange 424 of the leg 410. The flange 424 can also pivot relative to the arm 416 about an axis B along the axis of the leg 410 and fastener 422. The head 420 of the fastener 422 can be rotated to lock the flange 424 and the leg 410 relative to the arm 416. The interconnection of the arm 416, the leg 410 and the fastener 422 collectively form the pivotable/translational adjustment mechanism 408.

With continued reference to FIG. 6, the arm 416 can be substantially planar and include at a distal end a housing 426 forming a socket 428 for a ball 430 at a distal end of a connector 432. The socket 428 and the ball 430 form a universal (ball) joint of the universal rotational adjustment mechanism 406 for rotationally adjusting the connector 432 relative to the arm 416. After adjustment, the orientation of the connector 432 can be locked with a fastener 436 through the housing 426. The connector 432 supports the alignment rod 402 and includes an engagement surface 434 that can contact or engage the shaft 304, by a snap-on or other quick connect/disconnect connection. The support device 400 can be adjusted using the adjustment mechanisms 406, 408 described above such that the alignment rod 402 along axis A' is parallel to the alignment axis A of the shaft 304. In other words, the alignment rod 402 can serve as a marker for the orientation of the alignment axis A to guide reaming and cup insertion procedures as discussed below.

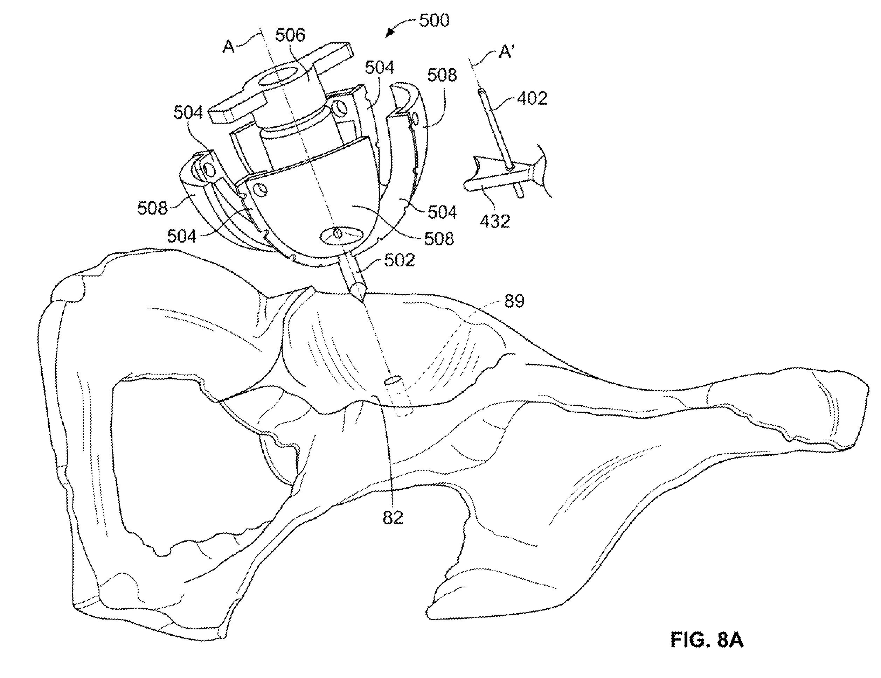

After the support device 400 is locked in a position such that the orientation of the alignment rod 402 along axis A' is fixed and parallel to the alignment axis A, the guiding handle 300 is disengaged from the engagement surface 434 of the connector 432 and the acetabular guide 100 and is removed. Referring to FIG. 7, a drilling element 440 can be guided through the bore 106 of the guiding element 104 of the acetabular guide 100 to drill a pilot hole 89 in the acetabulum 82 along the alignment axis A, as shown in FIG. 8A. The drilling element 440 can include a stop 442 at a pre-determined position to prevent over drilling or drilling through the wall of the acetabulum 82. The depth of drilling and the location of the stop 442 on the drilling element 440 can be determined during the pre-operative plan for the specific patient. The support device 400 and alignment rod 402 remain attached to the pelvis as shown in FIG. 6, although not fully shown in FIG. 7. After the pilot hole 89 is drilled, the acetabular guide 100 is removed.

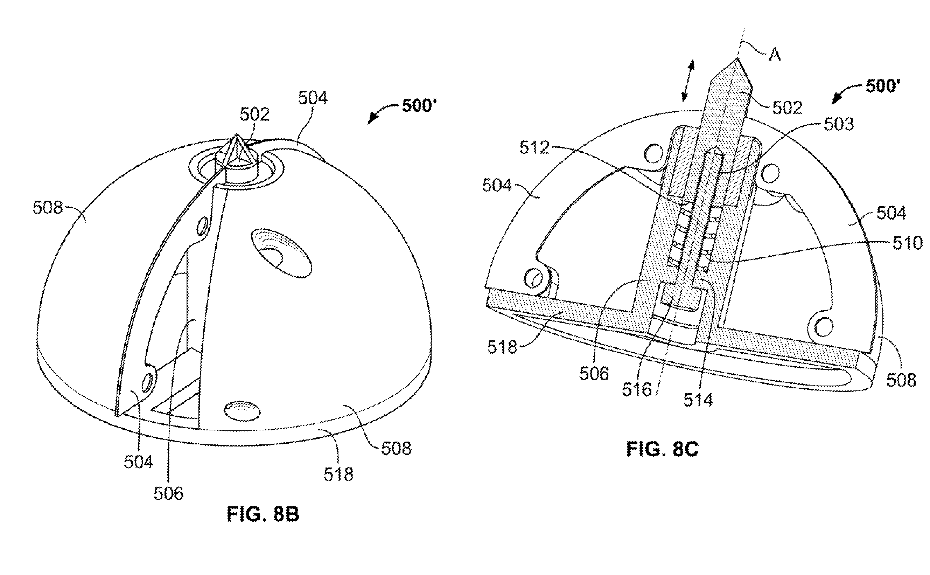

Referring to FIG. 8A, a reamer 500 can be guided along the alignment axis A to ream the acetabulum 82. Another embodiment of a reamer 500' according to the present teachings is illustrated in FIGS. 8B and 8C. The reamers 500 and 500' can be used interchangeably and similar elements will be referenced with the same numerals herein below. The reamer 500 (500') can include a trocar or other guiding pin 502 that is sized to fit and be received in the pilot hole 89 of the acetabulum 82 for stabilizing and guiding the reamer 500 (500') along the alignment axis A, i.e., at a predetermined location and orientation. This guided reaming arrangement enables the surgeon to recreate the preoperative planned position and orientation for reaming the acetabulum 82 and implanting the acetabular component. The alignment rod 402 which is supported by the support device 400 along the axis A' that is parallel to the alignment axis A can also help to guide the reamer 500 (500').

The reamer 500 (500') can include a plurality of curved reaming blades 504 and a supporting shaft 506 for a reamer driver or reamer handle. The curved blades 504 can be attached to a plurality of curved supporting elements 508 in the form of spherical leaves or spherical section/portions that collectively define a semi-spherical surface corresponding to the shape and size of the acetabular component to be implanted in the acetabulum after reaming. The blades 504 can be removable and replaceable or disposable. The entire reamer head that includes the blades 504 and the support element 508 can also be disposable. A reamer 500 with four disposable blades 504 is illustrated in FIG. 8A, while the reamer 500' shown in FIGS. 8B and 8C includes only two reamer blades 504. Referring to FIG. 8C, the guiding pin 502 can be spring biased to provide a tactile feedback during reaming. A spring or other biasing element 510 can be constrained between a proximal end 512 of the guiding pin 502 and a wall 514 of the supporting shaft 506. A set screw or fastener 516 can be used to stabilize the guiding pin 502 while allowing slidable movement along the alignment axis during reaming. The spring 510 can surround the fastener 516, as shown in FIG. 8C. Specifically, the fastener 516 is threaded to a blind bore 503 of the guiding pin 502 such that the fastener 516 and the guiding pin can move together along the alignment axis A by or against the action of the spring 510. The embodiments of FIGS. 8B and 8C also include a base ring 518 integrally attached to the shaft 506 providing additional stability.

Referring to FIG. 9, after the acetabulum 82 has been reamed, an acetabular inserter 550 can be coupled to an acetabular cup 280 by an end coupler 552 at the distal end of a shaft 554 of the acetabular inserter 550. As seen in FIG. 9, the shaft 554 can be slidably and removably coupled or engaged to the engagement surface 434 of the connector 432 of the support device 400, such that the shaft is oriented and can slide along the alignment axis A for insertion of the acetabular cup 280 according to the preoperatively planned position and orientation. In some embodiments, the acetabular inserter 550 can include an impact head 520 and can be used to impact the acetabular cup 280 after implantation.

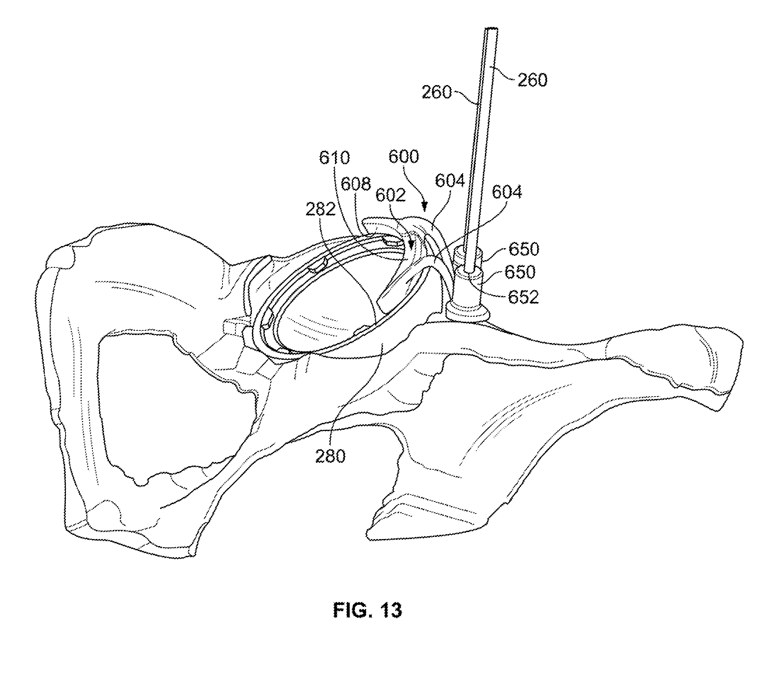

Referring to FIGS. 10-13, another method of reaming and preparing the acetabulum 82 is illustrated using the acetabular guides 200 with fitment options 200A and 200B, as described above in connection with FIGS. 4 and 5. In this method, markers pins 260 are inserted through the corresponding bores 252 of the marker elements 250 and attached to the bone in locations and orientations parallel to an axis B, as determined during the preoperative plan. The marker pins 260 can guide the location of a secondary guide 600, shown in FIG. 13, and designed according to the pre-operative plan to be guided by the marker pins 260, as discussed below.

The acetabular guide 200 can be slidably lifted off the marker pins 260 and removed, leaving the marker pins 260 attached to the bone. The acetabular cup 280 can be inserted using an acetabular inserter 550 without the aid of an alignment orientation, although a support device 400 with an alignment rod 402 can also be used if desired.

If desired, a reamer 500, 500' with a guiding pin 502 can be used to ream the acetabulum 82, as discussed above in connection with FIG. 7, although other commercially available reamers can also be used. As was described above in connection with FIG. 7 and the acetabular guides 100, a pilot hole 89 of predetermined length is drilled into the acetabulum 82 through the guiding element 204 with a drill element 440 until the stop 442 of the drilling element reaches the upper surface of the guiding element 204 of the acetabular guide 200.

After the acetabular cup 280 is inserted but not impacted, a secondary guide 600 having secondary marker elements 650 with bores 652 complementarily corresponding to the orientation and relative location of the marker elements 250 of the acetabular guide 200 is placed over the marker pins 260. The secondary guide 600 can be designed during the pre-operative plan such that the bores 652 are complementary to the location and orientation of the marker elements 250 of the acetabular guide. The secondary guide 600 can include extender elements 604 supporting an arcuate or crescent-shaped planar flange 602 having parallel inferior and superior surfaces 608, 610 designed during the pre-operative plan to be oriented parallel to a rim 282 of the acetabular cup 280, when the acetabular cup 280 is positioned in the predetermined position and orientation. The orientation and position of the acetabular cup 280 is adjusted using the secondary guide 600, such that the planar flange 602 (and the inferior and superior surfaces 608, 610 of the planar flange 602) and the rim 282 are parallel by ascertaining contact and engagement of the planar flange 602 to the rim 282 of the acetabular cup 280 in situ. It is noted that this method does not make use of the support device 400, although the acetabular guides 200 can also be used with the supporting device, at the discretion of the surgeon. Depending on the surgeons preferences, any selected or all the acetabular guides 100 (110A, 100B, 100C) and 200 (200A, 200B) and the associated instruments including the reamer 500, 500', the supporting device 400, the drilling element 440 with the stop 442, alignment rod 402, marker pins 260 and the secondary guide 600 can be provided in a surgical kit together with the acetabular cup 280 and/or additional implants and instruments.

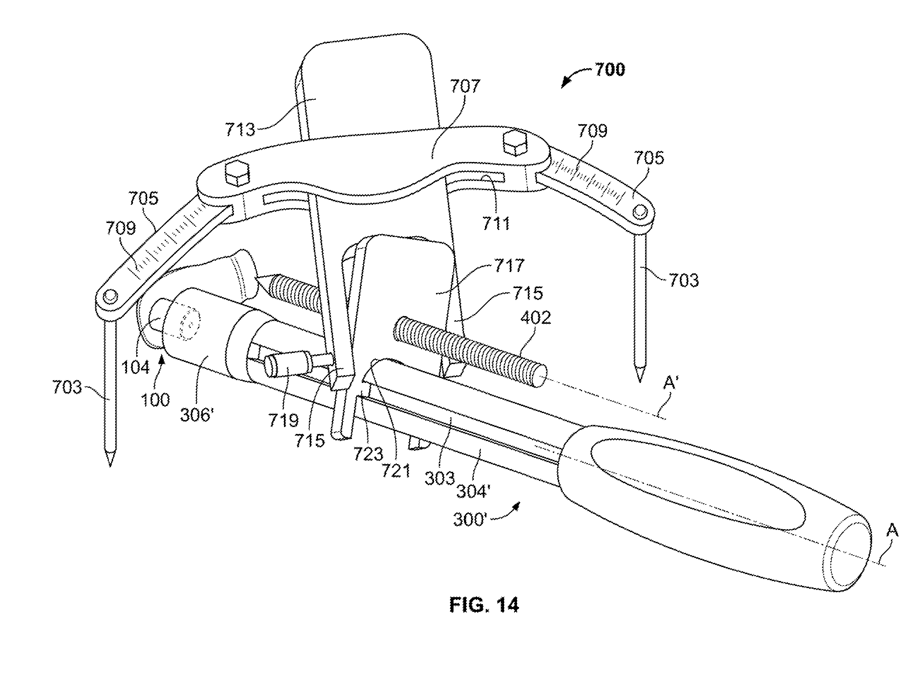

Referring to FIG. 14, another embodiment of the support device 400 is support device 700 illustrated with a guiding handle 300' and an acetabular guide 100 for positioning an alignment rod 402 in the pre-planned orientation A'. The support device 700 can be attached to the bone with bone pins 703 that pass through arms 705. The arms 705 extend slidably from a body 707 and can include scale markings 709 for measuring distances from the body 707. The body 707 can include a planar slot 711 through which a first planar flange 713 can slidably pass there through and locked with a fastener (not shown) after adjustment. The first planar flange 713 includes arms 715 pivotably connected to a second planar flange 717 along a pivot axis coinciding with a center axis of a locking element 719. The locking element 719 can be used to lock the relative orientations of the first and second planar flanges 713, 717. The second planar flange 717 can have a curved engagement surface 721 for slidably engaging a shaft 304' of the guiding handle 300'. The shaft 404' can include a groove 303 that is rotationally keyed to an element 721 extending from the second planar flange 717. The support device 700 can provide pivotable and translational adjustability to align the alignment rod 402 parallel to the alignment axis A which is fixed when the shaft 304' is attached to the guiding element 104 of the acetabular guide. The support device 700 can be used generally similarly to the support device 400 to provide a reference axis A' along the reference or alignment rod 402 to guide a reamer 500 or implant inserter 550.

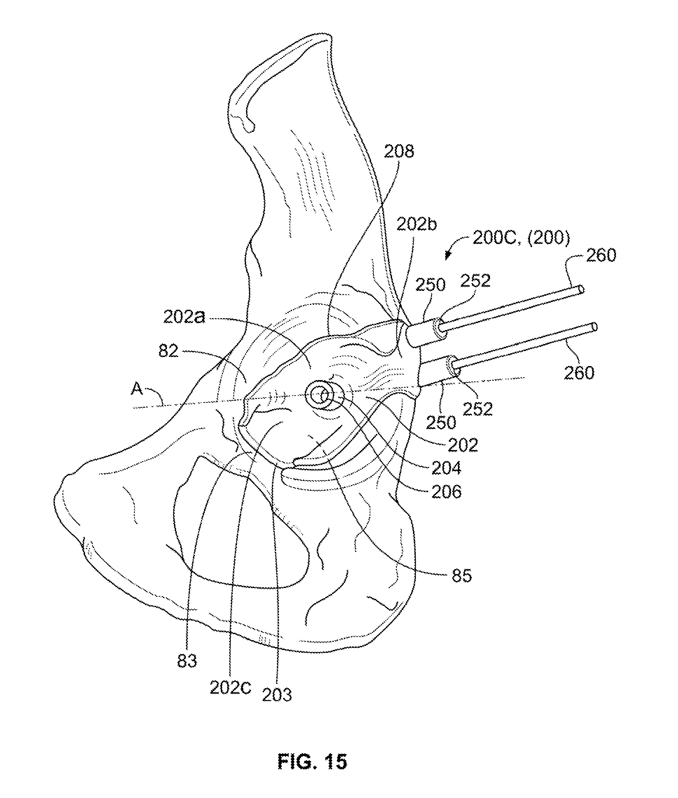

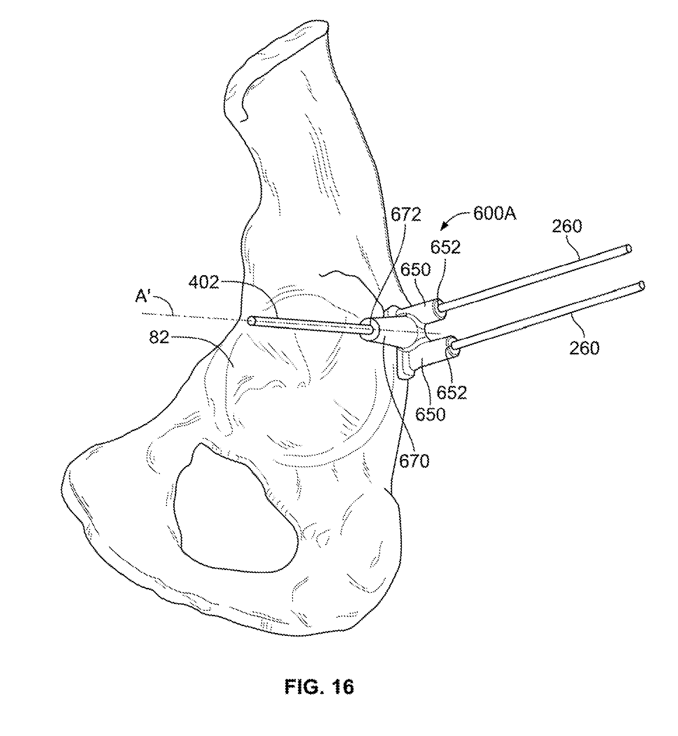

Referring to FIG. 15, another embodiment 200C of a patient-specific acetabular guide 200 is illustrated. Similarly to the embodiments illustrated in FIGS. 4 and 5 discussed above, the acetabular guide 200C includes a patient-specific body 202 and a guiding or pilot element 204 having an elongated bore 206 with an alignment axis A configured to be central to the acetabular cup and perpendicular to the acetabular cup's surface when the acetabular guide 200 is positioned on the acetabulum 82. The acetabular guide 200 can include one or more marker elements 250 (two are shown), each having an elongated bore 252 for guiding corresponding marker pins 260. The marker pins 260 can be used for supporting a secondary guide such as discussed below in reference to FIG. 12 above, or a secondary guide shown in FIG. 16.

The patient specific body 202 of acetabular guide 200C is similar to the body 202 of the acetabular guide 200A or 200B of FIGS. 4 and 5, and includes a first portion 202a from which the guiding element extends and which is designed to engage the acetabulum 82, and a second portion 202b which extends from the inner portion 202a and is configured to extend over a rim portion 84 of the acetabulum 82. The outer portion 202b extends sufficiently beyond the rim 84 to the periacetabular area of pelvis to accommodate the marker elements 250. The patient specific body 202 has an underside or bone-engaging three-dimensional engagement surface 208 that is custom-made or patient-specific to conform to and mirror (as a negative or inverse or mirror surface) complementary surfaces of selected portions of the acetabulum 82. In the embodiment of FIG. 15, the patient specific body 202 includes additionally a third portion 202c which conforms to and covers at least a portion of the acetabular fossa 85 of the acetabulum for providing additional rotational stability. Further, an inferior edge 203 of the acetabular guide 200C on the third portion 202c is designed during the preoperative plan to be parallel to the shape of the transverse acetabular ligament 83. The inferior edge 203 can provide an additional visual confirmation of correct alignment during positioning of the acetabular guide 200C on the acetabulum 82 of the patient.

Referring to FIG. 16, another embodiment of a secondary guide 600A is illustrated. The secondary guide 600A can be used with any of the acetabular guides 200 (200A, 200B, 200C). The secondary guide 600A can have first and second (secondary) marker elements 650 with bores 652. The secondary guide 600 can be designed during the pre-operative plan such that the bores 652 are complementary to the location and orientation of the marker elements 250 of the acetabular guide. The marker pins 260 are secured into the bone through the marker elements 250 of the acetabular guide 200, as shown, for example, in FIG. 10, and discussed above. The acetabular guide 200 is then lifted off the marker pins 260 and the secondary guide 600A is guided and placed over the marker pins 260. The secondary guide 600A is also designed during the preoperative plan for the patient to have a guiding element 670 with a longitudinal bore 672 along an axis A' that is parallel to the alignment axis A for the acetabular implant, when the secondary guide 600A is seated on the acetabulum over the marker pins 260, as shown in FIG. 16. Accordingly, an alignment pin 402 inserted into the bore 672 can serve as an indicator of the alignment direction A for reaming the acetabulum and inserting the acetabular cup.

As discussed above, the alignment axis A is the pre-operatively planned axis for insertion of the acetabular cup 280. Although the alignment axis A is shown, for simplicity, to coincide with the center axis of various instruments used for inserting, reaming and impacting the acetabular cup 280, it should be appreciated that this designation is adapted to indicate the final positioning of the instrument in the desired alignment. In other words, the alignment axis A and the axis of the instrument used are brought to coincidence by the methods described herein.

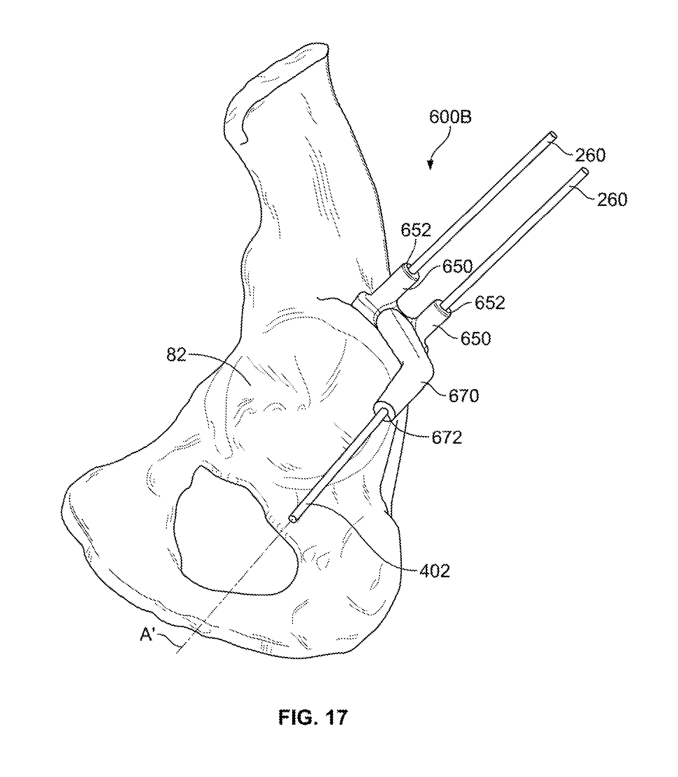

In some embodiments of the secondary guide, such as the secondary guide 600B shown in FIG. 17, the guiding element 670 may be placed in an offset position relative to the secondary marker elements 650 to provide better visualization of the acetabulum while guiding a reamer, inserter, impactor or other instrument along the alignment axis A parallel to the alignment pin 402.

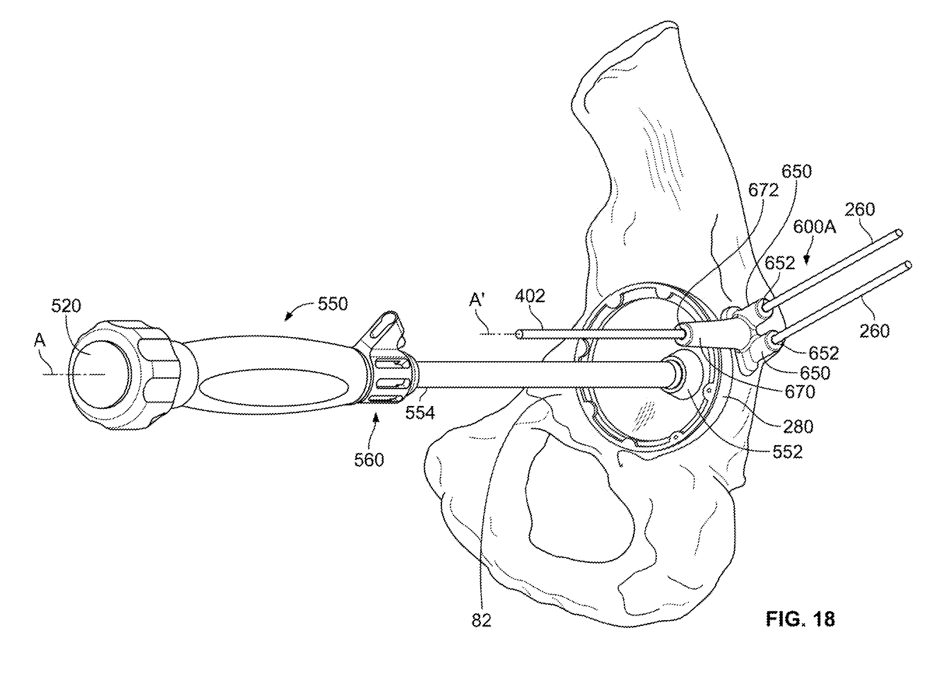

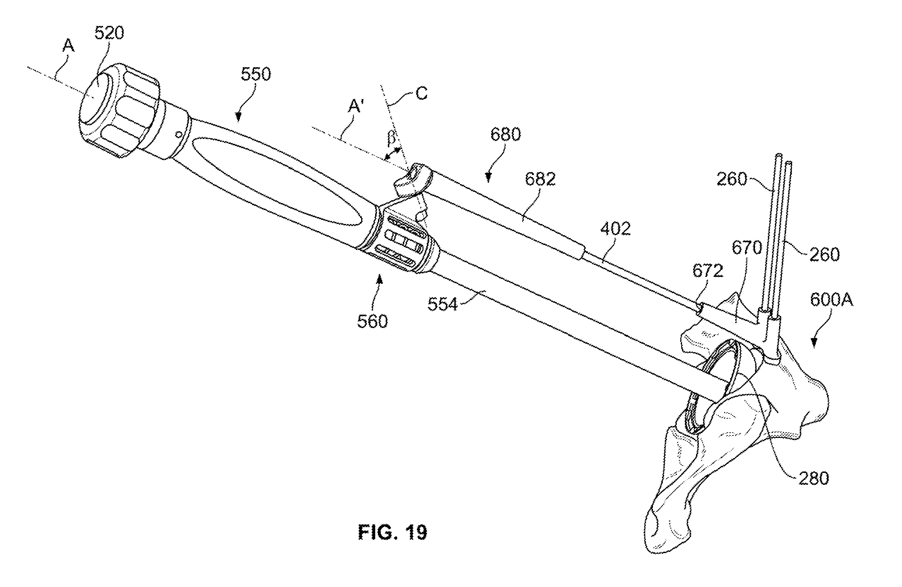

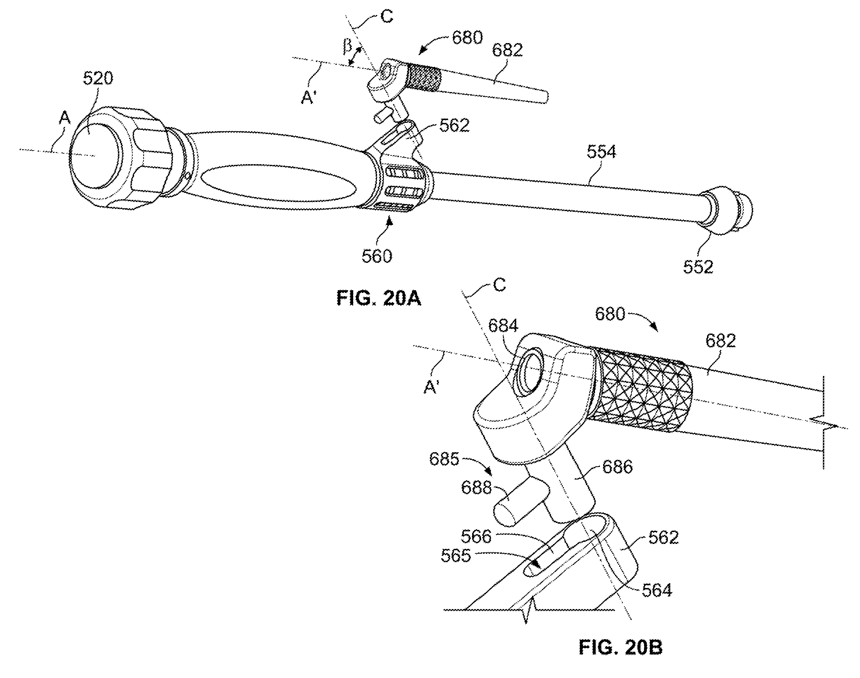

Referring to FIGS. 18-20B, the acetabular inserter 550 coupled to the acetabular cup 280 can be guided along the alignment axis A by the alignment pin 402 of the secondary guide 600A (or the offset secondary guide 600B), as an alternate method that does not use the support device 400 described above and shown in FIG. 9. In some embodiments, an adaptor 680, shown in FIGS. 19-20B, can be optionally used to couple the acetabular inserter 550 to the alignment pin 402 of the secondary guide 600A, as a further aid to orient the acetabular inserter 550 along the alignment axis A. In particular, the adaptor 680 includes an arm 682, such as a cannulated shaft having a longitudinal bore 684 that receives the alignment pin 402 when the adaptor 680 is placed over the alignment pin 402. Accordingly, the shaft 682 is aligned along the axis A' that is defined by the guiding element 670 of the secondary guide 600A. The adaptor is coupled to a rotatable collar 560 of the acetabular inserter 550 and secures the acetabular inserter 550 along the alignment axis A parallel to the axis A' of the shaft 682 and alignment pin 402. The adaptor 680 can be coupled to the collar 560 with a tongue-in-groove or other keyed connection, as illustrated in FIGS. 20A and 20B. In some embodiments, for example, the collar 560 can include an extension 562 forming a keyhole-shaped opening 565 with a first portion 564 having a circular cross-section and a second portion 566 having an elongated cross-section. The keyhole opening 565 receives correspondingly shaped portions of the adaptor 680, forming a key 685 for the keyhole opening 565 and preventing rotational movement. The key 685 has a first cylindrical element 686 received in the first portion 564 of the opening 565 and a second element 688 extending at a right angle from the first element 686 and received into the second portion 566 of the keyhole opening 565. Additionally, the key 685 and the keyhole opening 565 are angled at an acute angle relative to the alignment axis A and axis A'. Specifically, the first portion 564 of the keyhole opening 565 and the first element 686 of the key 685 are oriented along the direction of an axis C forming an acute angle β with axes A and A' as shown in FIGS. 20A and 20B. This skewed orientation facilitates the insertion of the key 685 into the keyhole opening 565 and the coupling of the adaptor 680 to the acetabular inserter 550.

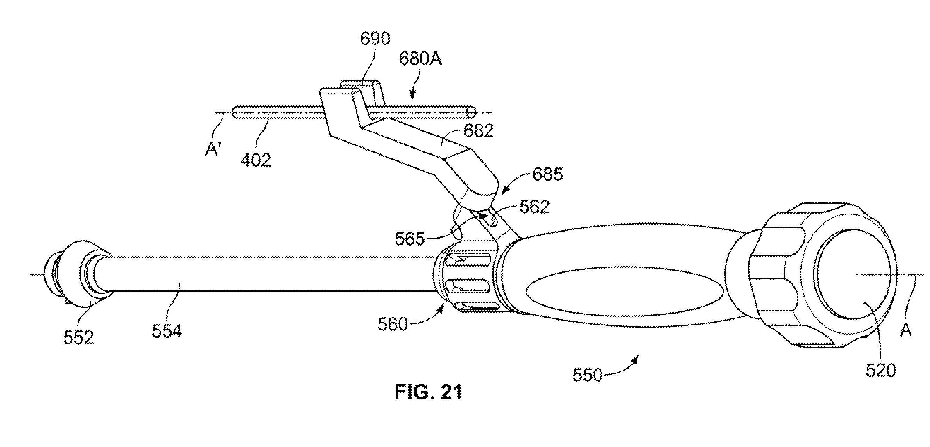

Referring to FIG. 21, in some embodiments 680A, the arm of 682 of the adaptor 680A can include an external V-shaped notch 690 instead of an internal cannulation 684 of the embodiment 680 of FIGS. 20A and 20B, while retaining the key 685 for engagement with the keyhole opening 565 of the acetabular inserter 550. The notch 690 can receive the alignment pin 402 in a semi-constrained arrangement for facilitating engagement and disengagement between the adaptor 680A and the alignment pin 402.

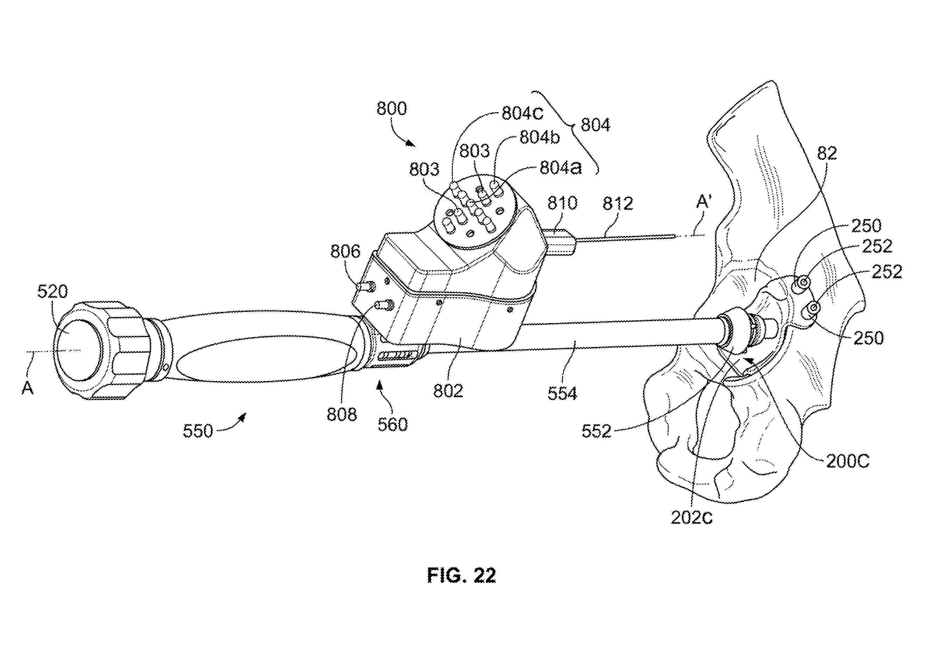

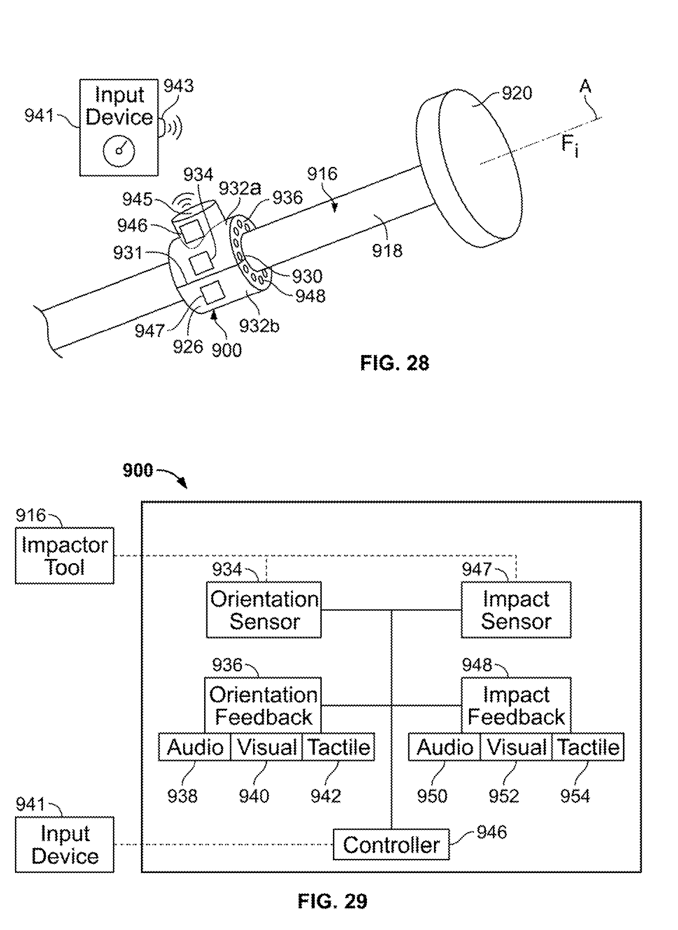

Referring to FIGS. 22-29, various methods for preparing the acetabulum for receiving an implant will be discussed in connection with the use of electronic positioners (800, 900) or other orientation devices for guiding the surgeon along the alignment axis A during reaming and/or inserting and impacting the acetabular implant into the acetabulum. Any commercially available orientation sensing devices can be used for the electronic positioner 800 (FIG. 22) or electronic positioner 900 (FIGS. 28 and 29). Various embodiments of the electronic positioner 900 are described in commonly assigned patent publications 2010/0249657, and 2010/0249796, both published on September 30, 2010. The electronic positioners can include a number of gyroscopic sensors and/or accelerometers that can measure deviations from a predetermined orientation and maintain or guide a return to that orientation. In the context of the present teachings, the predetermined orientation is the alignment axis A that was discussed above. Further, digital or MEMS gyroscopes of small size and capable of measuring deviation about two or three sin axes can be used, such as, for example, a three-axis, single sensor digital gyroscope (model L3G4200D) available from STMicroelectronics, Carrollton, Texas. It should be appreciated, however, that any combination of orientation sensors capable of identifying an orientation in three-dimensional space, including combinations of gyroscopic sensors (digital, analog, fiber optic or other type), having one or more axes, with other orientation sensors, such as accelerometers, such that the combination can identify deviations from an axis in three-dimensional space. For example, a 3-axis gyroscope (yaw, roll and pitch), or three one-axis gyroscopes, or one 2-axis gyroscope and one accelerometers, or three accelerometers or other combinations of gyroscopes and accelerometers. In some embodiments, the accelerometers can be single or multiple axes accelerometers. Mechanical, piezoelectric, MEMS, analog or digital accelerometers and gyroscope can be used. Additionally, a miniaturized compass, a laser, and/or GPS device may be included with the electronic positioner.

Referring to FIG. 22, the electronic positioner 800 can include a housing 802 that is removably attachable to a shaft of an inserter, impactor, reamer or other instrument used in connection with the acetabular arthroplasty or other corrective procedure. In FIG. 22, the electronic positioner is shown coupled to the acetabular inserter 550. The electronic positioner 800 can be, for example, keyed to the shaft 554, slidably attached to the shaft 554 or the collar 560 of the acetabular inserter 800 or slip-fit or with a clamp-on or tongue-and-groove or other coupling arrangement. The housing 802 can include a controller/processor communicably coupled to the orientation sensor/sensors discussed above, and a feedback module. An exemplary block diagram for the electronic positioner 900 of FIG. 20 is shown in FIG. 29, described below, and can be used for the electronic positioner 800, although the impact sensor and associated modules are optional and can be omitted from the electronic positioner of 800.

The electronic positioner 800 can include a power or on/off button 806, a set button/input device 808 for setting a pre-determined orientation during calibration and a set of LEDs 804 (outer set 804) for guiding the shaft 554 along the predetermined orientation (i.e., the alignment axis A) by lighting up to indicate a direction. The electronic positioner 800 can also include an optional laser device 810 capable of providing a reference orientation 812. The laser device 810 may be used, for example, with accelerometers that measure in two non parallel directions in a plane perpendicular to the reference orientation 812 of the laser. The electronic positioner 800 can be calibrated using one of the patient-specific acetabular guides described above, such as, for example, the acetabular guide 200C, shown in FIG. 22. The acetabular guide 200C can be positioned at a unique position on the patient's acetabulum, such that the guiding element 204 defines the alignment axis A, as designed during the preoperative plan. The shaft 554 of the acetabular inserter 550 can be slidably coupled to the guiding element 204 as described above in reference to FIG. 6, for example, such that the longitudinal axis of the shaft 554 coincides with the alignment axis A.

With the shaft 554 of the inserter 550 aligned along the alignment axis A, and the electronic positioner 800 coupled thereon, the set button/input device 808 can be pressed or otherwise activated to set or identify the alignment axis A as a reference axis, and store its orientation. As the shaft 554 is moved, deviations from the reference axis can be calculated by the sensors (see 934, FIG. 29) of the electronic positioner, processed by the controller (see 946, FIG. 29) of the electronic positioner and feedback provided by the LEDs 804 indicating the direction and increment of deviation. For example, the direction of deviation or, alternatively the direction to move to correct the deviation, can be indicated by the direction defined by a lit LED, such as, for example, 804b relative to a central LED 804a, which lights up when the shaft 554 is correctly aligned along the reference/alignment axis A. In some embodiments, a relative amount or degree of deviation for a given direction of a deviation can be indicated by a change in color of the LEDs 804, or by another set of LEDs 803 (inner set) that light up at a different fixed color, for example a fixed yellow to a fixed red of the outer set of LEDS 804. It will be appreciated that other types of feedback mechanisms or arrangements with or without LEDS and including visual, audio and vibratory or tactile feedback can be used, as disclosed in patent publications 2010/0249657 and 2010/0249796, referenced above, and further discussed below in reference to FIGS. 28 and 29.

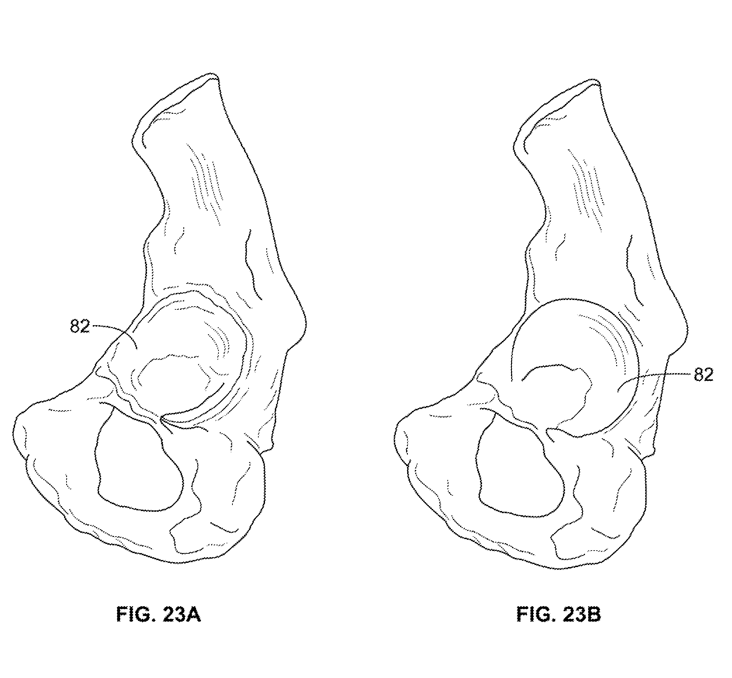

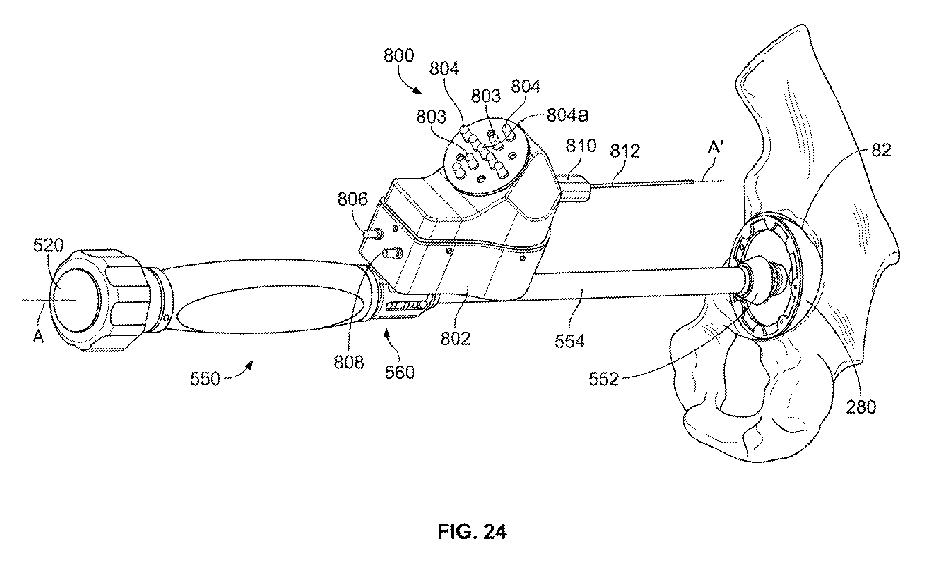

After the electronic positioner 800 is calibrated, the surgeon may ream the acetabulum 82 using a surgeon-selected method or any of the methods discussed above. See, for example, FIG. 8A and associated discussion. FIGS. 23A and 23B illustrate the patient's acetabulum 82 before and after reaming, respectively. After reaming, the acetabular inserter 500 with the electronic positioner 800 thereon can be coupled to the acetabular implant 280, as shown in FIG. 24, for inserting the acetabular implant 280 along the predetermined alignment axis A, as guided by the electronic positioner 800. Feedback from the LEDs 804 and/or 803 or other feedback mechanism can help guide the surgeon to correct any deviation from the predetermined alignment axis A that coincides with the calibrated reference axis of the electronic positioner 800, as discussed above.

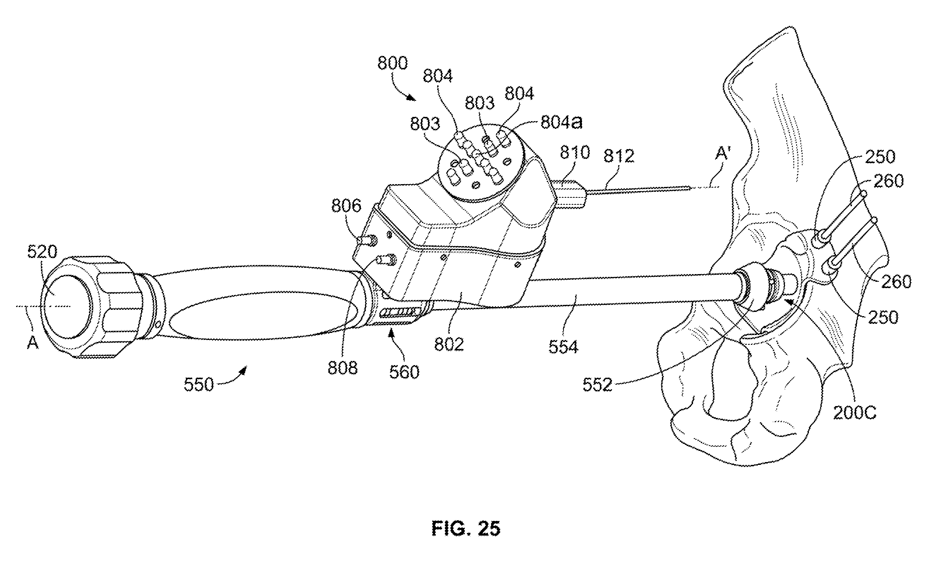

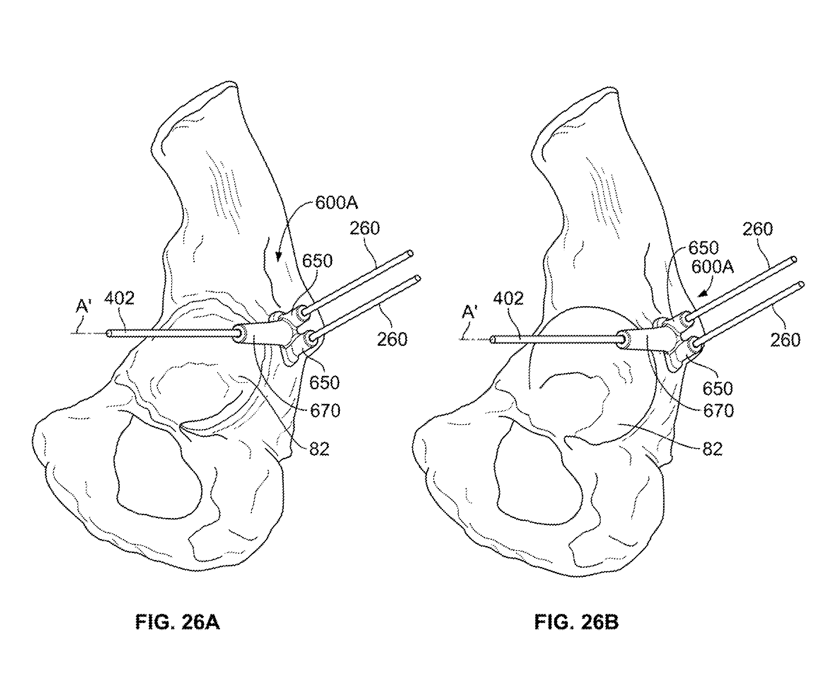

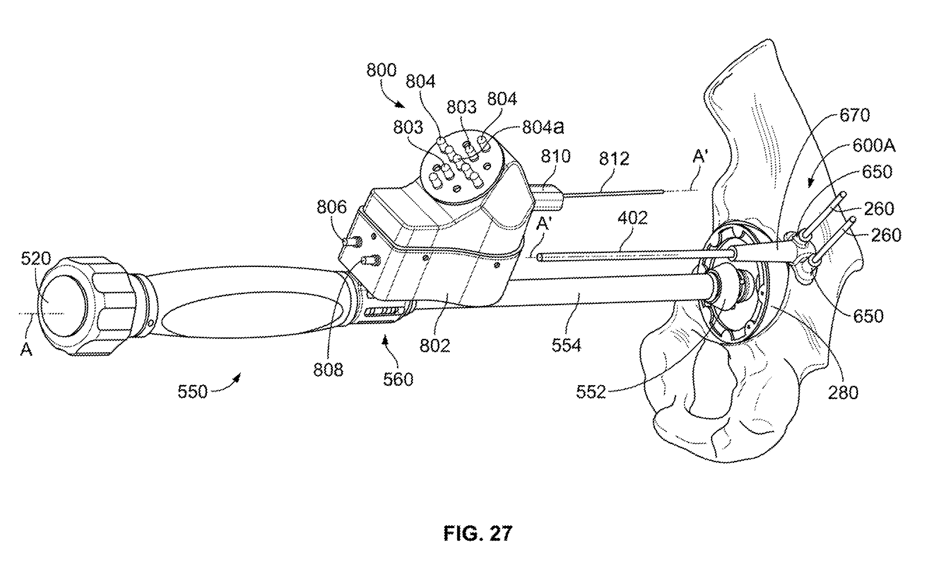

When using the electronic positioner 800, as described above, marker pins 260 and a secondary guide 600, 600A, 600B are not necessary. Depending on the surgeon's preference, when additional guidance is desired, the marker pins 260 and the secondary guide 600A (or 600 or 600B) can be used together with the electronic positioner 800, as illustrated in FIGS. 25-27. The marker pins 260 can be inserted, for example, through the marker elements 250 of the patient-specific acetabular guide 200C, as shown in FIG. 25 during calibration of the electronic positioner 800. After calibration, the patient-specific acetabular guide 200C is lifted off the marker pins 260, which are left attached to the acetabulum 82 for guiding the placement of the secondary guide 600A (or 600B or 600), as shown in FIG. 26A. The alignment pin 402 can be used as a reference for the implant alignment axis A. In some embodiments, the alignment pin 402 can be used to align a supporting shaft 506 of a reamer 500 (see FIG. 8A) parallel to the direction A' of the alignment pin 402 and used during reaming. After reaming, the secondary guide 600A and the marker pins 260 can remain attached to the acetabulum, as shown in FIG. 26B, to further guide the surgeon during the insertion of the acetabular cup 280. Accordingly, and referring to FIG. 27, the alignment pin 402 of the secondary guide 600A can provide an initial reference direction A' for the surgeon to position the acetabular inserter 800 easier and quicker along the alignment direction A, and make smaller corrections in orientation with the help of the LEDs 804, 803 or other feedback mechanism of the electronic positioner 800.

Referring to FIGS. 28 and 29, the electronic positioner 900 of the above referenced patent publications 2010/0249657 and 2010/0249796 is briefly described. It will be appreciated that in some embodiments, the electronic positioner 900 can replace the electronic positioner 800 in the methods described above. In other embodiments, the electronic positioner 800 can incorporate all or some of the functions of the electronic positioner 900, while maintaining, for example, the geometry of the housing 802 and the LED feedback arrangement described above in reference to FIG. 22. In some embodiments, the electronic positioner 800 can include a orientation sensor (or sensors) 934, but not impact sensors 947 and impact feedback, as discussed below. In other embodiments, the electronic positioner 800 may also include the impact sensor 947 and associated feedback mechanisms of the electronic positioner 900.

As shown in FIGS. 28 and 29, the electronic positioner 900 can include a cylindrical housing 926 coupled to an acetabular inserter/impactor tool 916 having a longitudinal shaft 918. Specifically, the housing 926 can include a passage 930 that receives the shaft 918 and couples the housing 926 to the acetabular inserter/impactor tool 916 in a fixed position relative to the shaft 918. The housing 926 can include a seam or hinge or other longitudinal separator 931 between first and seconds portions 932a, 932b of the housing 926 for removing the housing 926 from the shaft 918. In some embodiments, the hinge 931 can operate to secure the first and second portions 932a, 932b in a clam shell design of the housing 926.

It will be appreciated that the housing 926 can be attached to the shaft 918 in any suitable fashion, such as an interference fit, a taper to taper fit between the shaft 918 and the inner surface of the passage 930, or other arrangement. In some embodiments, the shaft 918 can include a recess (not shown) that receives the housing 926 such that the housing 926 is in a fixed position relative to the shaft 918. Further, the electronic positioner 900 can be removably coupled to various commercially available inserter/impactor tools 916, such as a RingLoc^{®} Inserter (Part No. S313141) or a Magnum Inserter (Part No. 313131), commercially available from Biomet Manufacturing Corp., of Warsaw, Indiana. The electronic positioner 900 can be similarly coupled to the shaft of the inserter 550 or reamer 500 or guiding handle 300 or other tool used during the acetabular procedure for guiding along the alignment axis A.

As shown in FIGS. 28 and 29, the electronic positioner 900 can include one or more orientation sensors 934. The orientation sensor 934 can detect an actual orientation of the longitudinal axis of the inserter/impactor tool 916 relative to a reference orientation or reference vector, such as the force of gravity. In some embodiments, the orientation sensor 934 can detect acceleration about three separate orthogonal axes (accelerometer sensors) or angular velocity (spin) about one or more orthogonal axes (gyroscopic sensor), as discussed above in reference to the electronic positioner 800.

The electronic positioner 900 (or 800) can include an orientation feedback device 936. The orientation feedback device 936 can selectively provide an orientation feedback signal when the actual orientation of the inserter/impactor tool 916 is substantially equal to a predetermined target orientation, i.e., the alignment axis A discussed above. The feedback signal provided by the orientation feedback device 936 can automatically indicate to the surgeon that the inserter/impactor tool 916 is in the target orientation, such that the acetabular cup can be properly positioned and implanted for added convenience and accuracy.

As represented in FIG. 29, the orientation feedback device 936 can include an audible feedback device 938 that emits an audible feedback signal. For instance, the audible feedback device 938 can include a speaker that emits a preprogrammed sound when the inserter/impactor tool 916 is in the target orientation. Furthermore, the orientation feedback device 936 can include a visual feedback device 940 that emits a visual feedback signal. For instance, the visual feedback device 940 can include one or more lights, such as LED lights, for emitting a preprogrammed light pattern when the inserter/impactor tool 916 is in the target orientation. An embodiment of the visual feedback device 940 is described above in connection with the LEDs 804 and 803 of the electronic positioner 800. Additionally, the orientation feedback device 936 can include a tactile feedback device that selectively emits a tactile feedback signal when the inserter/impactor tool 916 is in the target orientation. For instance, the tactile feedback device 942 can include a vibration motor that selectively vibrates the housing 926 and the inserter/impactor tool 916 when the inserter/impactor tool 916 is in the target orientation.

It will be appreciated that the orientation feedback device 936 can provide any suitable feedback signal, including combinations of visual, audible and tactile feedback. Also, it will be appreciated that the feedback signal can be seen, heard, and felt simultaneously, and this redundancy can increase accuracy and convenience.

The electronic positioner 900 (or 800) can include a controller 946. The controller 946 can include various components, such as a microprocessor, memory, and the like. The controller 946 can be in communication with the orientation sensor 934 and the orientation feedback device 936. Accordingly, the controller 946 can cause the orientation feedback device 936 to selectively provide the respective orientation feedback signal(s) when the actual orientation of the inserter/impactor tool 916 detected by the orientation sensor 934 is substantially equal to a predetermined target orientation of the inserter/impactor tool 916.

The electronic positioner 900 (or 800) can include an impact sensor 947 to help the surgeon determine more accurately and conveniently when the acetabular cup 280 is fully seated in the acetabulum 82 of the patient. The impact sensor 947 can detect an actual impact effect on the inserter/impactor tool 916. For instance, the impact sensor 947 can be configured to detect an actual impact force Fᵢ on the head 920 of the inserter/impactor tool 916. The impact sensor 947 can also detect an actual displacement of the inserter/impactor tool 916 when the load Fᵢ is applied to the head 920. In addition, the impact sensor 947 can detect an actual acoustic effect of the impact when the load Fᵢ is applied. It will be appreciated that the impact sensor 947 can be configured to detect any suitable effect of applying the load Fᵢ. The impact sensor 947 can include any suitable component, such as an accelerometer and/or a piezoelectric sensor, for detecting the actual impact effect. It will be appreciated that the orientation sensor 934 and the impact sensor 947 can rely, at least in part, on some of the same components, such as a common accelerometer, for the respective functions.

With continued reference to FIGS. 28 and 29, the electronic positioner 900 (or 800) can include an impact feedback device 948. Generally, the impact feedback device 948 can provide a feedback signal when the actual effect of applying the load Fᵢ detected by the impact sensor 947 substantially matches a predetermined target impact effect. More specifically, the surgeon can predetermine a target impact effect (e.g., a predetermined impact force Fᵢ, an amount of displacement, an acoustic effect, etc.) that correlates to a condition in which the acetabular cup 280 is fully seated in the acetabulum 82 of the patient. Then, the controller 946 can cause the impact feedback device 948 to provide the respective feedback signal when the actual impact effect substantially matches the predetermined target impact effect. Thus, the impact feedback device 948 can inform the surgeon that the acetabular cup 280 has been fully seated into the acetabulum 82 of the patient.

It will be appreciated that the impact feedback device 948 can include any suitable device, such as an audible feedback device 950, a visual feedback device 952, and/or a tactile feedback device 954. The audible feedback device 950 can provide an audible feedback signal, and can include a speaker or any other suitable device for providing an audible feedback signal. The visual feedback device 952 can provide a visual feedback signal, and can include one or more lights, such as LED lights, for providing a visual feedback signal. Also, the tactile feedback device 954 can provide a tactile feedback signal and can include a vibration motor that selectively provides a tactile feedback signal.

In some embodiments, the electronic positioner 900 (or 800) can include an input device 941 for manually setting the target orientation of the inserter/impactor tool 916. More specifically, the input device 941 can include buttons, dials, a display, and other features for setting a target inclination angle, a target anteversion angle, or any other setting for the orientation of the alignment axis A. The input device 41 can be integrally coupled to the housing 926, and/or the input device 941 can be separate and remote from the housing 926. For example, as shown in FIG. 28, the input device 941 can include a wireless transmitter 943, and the housing 926 can include a wireless receiver 945. The receiver 945 receives wireless signals from the transmitter 943 to thereby set the target orientation of the alignment axis A. In some embodiments, the input device 941 can include an alphanumeric keypad for manually inputting and setting a particular target orientation. Additional details about of the electronic positioner 900 can be found in patent publications 2010/0249657 and 2010/0249796, referenced above.

Various patient-specific guides, secondary guides, reamers, guide handles, inserters, impactors, support devices, electronic positioners and other instruments can be used in various combinations and based on surgeon preferences or patient and preoperative or intraoperative circumstances for preparing an acetabulum and guiding and implanting an acetabular implant along a preoperatively determined alignment orientation. In this respect, tools and instrumentation providing redundant functionality and of different embodiments may provided to the surgeon in a kit or per surgeon's request. For example, more than one patient specific acetabular guide (100, 200) may be provided. Similarly, various support devices (400, 700), secondary guides (600, 600A, 600B), reamers (500, 500'), inserters and/or impactors (550, 916), adaptors or couplers, electronic positioners (800, 900) and other instruments described above can be provided and used in various combinations within the methods described herein.

The foregoing discussion discloses and describes merely exemplary arrangements of the present teachings. Furthermore, the mixing and matching of features, elements and/or functions between various embodiments is expressly contemplated herein, so that one of ordinary skill in the art would appreciate from this disclosure that features, elements and/or functions of one embodiment may be incorporated into another embodiment as appropriate, unless described otherwise above. Moreover, many modifications may be made to adapt a particular situation or material to the present teachings without departing from the essential scope thereof. One skilled in the art will readily recognize from such discussion, and from the accompanying drawings and claims, that various changes, modifications and variations can be made therein without departing from the scope of the present teachings as defined in the following claims.

## Claims

1. An acetabular guide (200, 200A, 200B, 200C) comprising:
a patient-specific engagement surface designed to be complementary and mateable with a corresponding surface of the patient's pelvic anatomy as determined during a pre-operative plan of the patient by a three-dimensional reconstruction of the anatomy of the patient using two-dimensional medical images, the patient-specific engagement surface having a first portion mateable with a portion of an acetabulum of the patient;
a guiding element (204) extending from the acetabular guide opposite to the first portion of engagement surface, the guiding element defining a bore designed to be oriented along an alignment axis for an acetabular implant when the acetabular guide is engaged to the acetabulum;
first and second marker elements (250) extending from a portion of the acetabular guide outside the acetabulum of the patient, the marker elements defining corresponding first and second bores (252) for guiding first and second marker pins (260) into a bone portion of the patient in combination with
a secondary guide (600A, 600B) designed during the pre-operative plan to include first and second secondary marker elements (650) complementary to the first and second marker elements of the acetabular guide for receiving the first and second marker pins (260) when the first and second marker pins are attached to a bone portion of the patient, **characterised in that** the secondary guide includes
a second guiding element (670), the second guiding element defining a bore (672) designed to be oriented parallel to the alignment axis for the acetabular implant when the secondary guide is engaged to the acetabulum.

2. The combination of claim 1, wherein the engagement surface includes a second portion mateable with at least one of a portion of a rim of the acetabulum of the patient, a portion of an acetabular fossa of the patient, and a portion of a transverse acetabular ligament of the patient.

3. The combination of claim 1, wherein the secondary guide (600A, 600B) includes a planar arcuate surface complementary to a rim of an acetabular cup, the planar arcuate surface designed during the preoperative plan to orient the acetabular cup in a pre-planned orientation and location in the acetabulum relative to an alignment axis when the planar arcuate surface of the secondary guide engages the rim of the acetabular cup.

4. The combination of claim 1 in combination with:
a guiding handle (300) removably attachable to the guiding element of the acetabular guide along the alignment axis; and
a support device attachable to a pelvic portion of the patient, the support device including a connector supporting an alignment rod, the connector removably engageable with a shaft of the guiding handle, the support device including rotational and translational mechanisms for orienting the alignment rod parallel to the shaft of the guiding handle and parallel to the alignment axis.

5. The combination of claim 4, further comprising an inserter (550) for an acetabular cup, the inserter including a shaft engageable with the connector to orient the shaft along the alignment axis for inserting the acetabular cup into the acetabulum of the patient.

6. The combination of claim 1 in combination with a reamer (500, 500') for reaming the acetabulum for receiving an acetabular cup, the reamer including a guiding pin receivable in a pilot hole oriented along the alignment axis and drilled in the acetabulum through the bore of the guiding element.

7. The combination of claim 6, wherein the reamer includes a spring that biases the guiding pin and provides tactile feedback during reaming.

8. The combination of claim 6, wherein the reamer includes a plurality of removable arcuate blades, wherein each blade is attachable to a corresponding supporting element shaped as a spherical section, such that the supporting elements collectively form a surface corresponding to a shape of the acetabular cup.

9. The combination of claim 1, wherein the second guiding element is offset relative to the first and second secondary marker elements of the secondary guide.

10. The combination of claim 1, further comprising:
an alignment pin receivable in the bore of the guiding element;
an adaptor mountable over the alignment pin; and
an acetabular inserter (550) including a collar (560) configured to engage the adaptor and orient a shaft of the acetabular inserter along the alignment axis.

11. The combination of claim 1, wherein the engagement surface includes an interior edge configured to be parallel and conforming to a portion of a transverse acetabular ligament of the patient.

12. The combination of claim 1 in combination with:
an acetabular cup inserter; and
an electronic positioner (800, 900) removably attached to the shaft of the acetabular inserter, the electronic positioner capable of indicating a predetermined orientation based on calibration with the acetabular guide.

## Patentansprüche

1. Hüftführung (200, 200A, 200B, 200C), umfassend:
eine patientenspezifische Eingriffsfläche, die ausgebildet ist, um mit einer entsprechenden Fläche der Beckenanatomie des Patienten ergänzt und angepasst werden zu können, wie bei einem präoperativen Plan des Patienten durch eine dreidimensionale Rekonstruktion der Anatomie des Patienten unter Verwendung zweidimensionaler medizinischer Bilder bestimmt, wobei die patientenspezifische Eingriffsfläche einen ersten Abschnitt aufweist, der mit einem Abschnitt eines Acetabulum des Patienten zusammengepasst werden kann;
ein Führungselement (204), das sich aus der Hüftführung gegenüber des ersten Abschnitts der Eingriffsfläche erstreckt, wobei das Führungselement eine Bohrung definiert, die ausgebildet ist, um entlang einer Ausrichtungsachse für ein Hüftimplantat orientiert zu sein, wenn die Hüftführung im Eingriff mit dem Acetabulum ist;
erste und zweite Markierungselemente (250), die sich aus einem Abschnitt der Hüftführung außerhalb des Acetabulums des Patienten erstrecken, wobei die Markierungselemente entsprechende erste und zweite Bohrungen (252) zum Führen erster und zweiter Markierungsstifte (260) in einen Knochenabschnitt des Patienten definieren, in Kombination mit einer sekundären Führung (600A, 600B), die während des präoperativen Plans ausgebildet wird, um erste und zweite sekundäre Markierungselemente (650) einzuschließen, die die ersten und zweiten Markierungselemente der Hüftführung ergänzen, um die ersten und zweiten Markierungsstifte aufzunehmen (260), wenn die ersten und zweiten Markierungsstifte an einem Knochenabschnitt des Patienten angebracht sind, **dadurch gekennzeichnet, dass** die sekundäre Führung
ein zweites Führungselement (670) einschließt, wobei das zweite Führungselement eine Bohrung definiert (672), die ausgebildet ist, um parallel zu der Ausrichtungsachse des Hüftimplantats orientiert zu sein, wenn die sekundäre Führung im Eingriff mit dem Acetabulum ist.

2. Kombination nach Anspruch 1, wobei die Eingriffsfläche einen zweiten Abschnitt einschließt, der mit zumindest einem von einem Abschnitt eines Randes des Acetabulums des Patienten, einem Abschnitt eines Pfannenbodens des Patienten und einem Abschnitt eines Ligamentum transversum acetabuli zusammengepasst werden kann.

3. Kombination nach Anspruch 1, wobei die sekundäre Führung (600A, 600B) eine ebene, bogenförmige Fläche einschließt, die einen Rand einer Hüftgelenkpfanne ergänzt, wobei die ebene, bogenförmige Fläche während des präoperativen Plans ausgebildet wurde, um die Hüftgelenkpfanne in einer vorausgeplanten Orientierung und Lage in dem Acetabulum bezogen auf eine Ausrichtungsachse zu orientieren, wenn die ebene, bogenförmige Fläche der sekundären Führung in den Rand der Hüftgelenkpfanne eingreift.

4. Kombination nach Anspruch 1, in Kombination mit:
einem Führungsholm (300), der lösbar an dem Führungselement der Hüftführung entlang der Ausrichtungsachse angebracht ist; und
einer Stützvorrichtung, die an einem Beckenabschnitt des Patienten angebracht werden kann, wobei die Stützvorrichtung einen Verbinder einschließt, der eine Ausrichtungsstange stützt, wobei der Verbinder lösbar an dem Schaft des Führungsholmes eingreifen kann, wobei die Stützvorrichtung Dreh- und Translationsmechanismen zum Orientieren der Ausrichtungsstange parallel zu dem Schaft des Führungsholmes und parallel zu der Ausrichtungsachse einschließt.

5. Kombination nach Anspruch 4, die ferner einen Einsatz (550) für eine Hüftgelenkpfanne umfasst, wobei der Einsatz einen Schaft einschließt, der an dem Verbinder eingreifen kann, um den Schaft zum Einsetzen der Hüftgelenkpfanne in das Acetabulum des Patienten entlang der Ausrichtungsachse zu orientieren.

6. Kombination nach Anspruch 1, in Kombination mit einer Reibahle (500, 500') zum Ausreiben des Acetabulums zum Aufnehmen einer Hüftgelenkpfanne, wobei die Reibahle einen Führungsstift einschließt, der in einem Führungsloch aufgenommen werden kann, das entlang der Ausrichtungsachse orientiert ist und durch die Bohrung des Führungselements in das Acetabulum gebohrt ist.

7. Kombination nach Anspruch 6, wobei die Reibahle eine Feder einschließt, die den Führungsstift vorspannt und Tastrückmeldung während des Ausreibens bereitstellt.

8. Kombination nach Anspruch 6, wobei die Reibahle eine Vielzahl von lösbaren bogenförmigen Messern einschließt, wobei jedes Messer an einem entsprechenden Stützelement angebracht werden kann, das als ein Kugelabschnitt geformt ist, sodass die Stützelemente zusammen eine Fläche bilden, die einer Form der Hüftgelenkpfanne entspricht.

9. Kombination nach Anspruch 1, wobei das zweite Führungselement bezogen auf die ersten und zweiten sekundären Markierungselemente der sekundären Führung versetzt ist.

10. Kombination nach Anspruch 1, ferner umfassend:
einen Ausrichtungsstift, der in der Bohrung des Führungselements aufgenommen werden kann;
einen Adapter, der über dem Ausrichtungsstift befestigt werden kann, und
einen Hüfteinsatz (550), einschließlich eines Kragens (560), der konfiguriert ist, um an dem Adapter einzugreifen und einen Schaft des Hüfteinsatzes entlang einer Ausrichtungsachse zu orientieren.

11. Kombination nach Anspruch 1, wobei die Eingriffsfläche eine Innenkante einschließt, die konfiguriert ist, um parallel und entsprechend eines Abschnitts eines Ligamentum transversum acetabuli des Patienten zu sein.

12. Kombination nach Anspruch 1, in Kombination mit:
einem Hüftgelenkpfanneneinsatz; und
einem elektronischen Positionierer (800, 900), der lösbar an dem Schaft des Hüftgelenkpfanneneinsatzes angebracht ist, wobei der elektronische Positionierer eine vorbestimmte Orientierung auf Grundlage von Kalibrierung mit der Hüftführung anzeigen kann.

## Revendications

1. Guide acétabulaire (200, 200A, 200B, 200C) comprenant :
une surface d'engagement spécifique à un patient conçue pour être complémentaire et accouplable à une surface correspondante de l'anatomie pelvienne du patient telle que déterminée lors d'un plan pré-opératoire du patient par une reconstruction en trois dimensions de l'anatomie du patient à l'aide d'image médicales en deux dimensions, la surface d'engagement spécifique au patient possédant une première partie accouplable à une partie d'un acétabulum du patient ;
un élément de guidage (204) se déployant depuis le guide acétabulaire en face de la première partie de la surface d'engagement, l'élément de guidage définissant un alésage conçu pour être orienté le long d'un axe d'alignement pour un implant acétabulaire quand le guide acétabulaire est en prise avec l'acétabulum ;
des premier et deuxième éléments marqueurs (250) se déployant depuis une partie du guide acétabulaire en dehors de l' acétabulum du patient, les éléments marqueurs définissant des premier et deuxième alésages (252) correspondants pour guider des premier et deuxième broches de marquage (260) dans une partie d'os du patient en combinaison avec un guide secondaire (600A, 600B) conçu lors du plan pré-opératoire pour inclure des premier et deuxième éléments marqueurs secondaires (650) complémentaires aux premier et deuxième éléments marqueurs du guide acétabulaire pour recevoir les première et deuxième broches de marquage (260) quand les première et deuxième broches de marquage sont attachées à une partie d'os du patient, **caractérisé en ce que** le guide secondaire inclut
un deuxième élément de guidage (670), le deuxième élément de guidage définissant un alésage (672) conçu pour être orienté parallèle à l'axe d'alignement pour l'implant acétabulaire quand le guide secondaire est en prise avec l'acétabulum.

2. Combinaison selon la revendication 1, dans laquelle la surface d'engagement inclut une deuxième partie accouplable avec au moins l'une d'une partie d'un rebord de l'acétabulum du patient, une partie de fosse acétabulaire du patient, et une partie d'un ligament transverse acétabulaire du patient.

3. Combinaison selon la revendication 1, dans laquelle le guide secondaire (600A, 600B) inclut une surface arquée planaire complémentaire à un rebord d'une cupule acétabulaire, la surface arquée planaire conçue lors du plan pré-opératoire pour orienter la cupule acétabulaire dans une orientation et localisation préalablement planifiées dans l'acétabulum par rapport à un axe d'alignement quand la surface arquée planaire du guide secondaire vient en prise avec le rebord de la cupule acétabulaire.

4. Combinaison selon la revendication 1 associée à :
une poignée de guidage (300) attachable de manière amovible à l'élément de guidage du guide acétabulaire le long de l'axe d'alignement ; et
un dispositif de support attachable à une partie pelvienne du patient, le dispositif de support incluant un connecteur supportant une tige d'alignement, le connecteur venant en prise de manière amovible avec un arbre de la poignée de guidage, le dispositif de support incluant des mécanismes de rotation et translation destinés à orienter la tige d'alignement parallèle à l'arbre de la poignée de guidage et parallèle à l'axe d'alignement.

5. Combinaison selon la revendication 4, comprenant en outre un insert (550) pour une cupule acétabulaire, l'insert incluant un arbre venant en prise avec le connecteur pour orienter l'arbre le long de l'axe d'alignement pour insérer la cupule acétabulaire dans l'acétabulum du patient.

6. Combinaison selon la revendication 1 associée à un alésoir (500, 500') destiné à aléser l'acétabulum pour recevoir une cupule acétabulaire, l'alésoir incluant une broche de guidage réceptionnable dans un orifice pilote orienté le long de l'axe d'alignement et foré dans l'acétabulum à travers l'alésage de l'élément de guidage.

7. Combinaison selon la revendication 6, dans laquelle l'alésoir inclut un ressort qui rappelle la broche de guidage et fournit une réaction tactile pendant l'alésage.

8. Combinaison selon la revendication 6, dans laquelle l'alésoir inclut une pluralité de lames arquées amovibles, dans laquelle chaque lame est attachable à un élément de support correspondant formé en une section sphérique, de manière à ce que les éléments de support forment collectivement une surface correspondant à une forme de la cupule acétabulaire.

9. Combinaison selon la revendication 1, dans laquelle le deuxième élément de guidage est décalé par rapport aux premier et deuxième éléments marqueurs secondaires du guide secondaire.

10. Combinaison selon la revendication 1, comprenant en outre :
une broche d'alignement réceptionnable dans l'alésage de l'élément de guidage ;
un adaptateur fixable sur la broche d'alignement ; et
un insert acétabulaire (550) incluant un collier (560) configuré pour venir en prise avec l'adaptateur et orienter un arbre de l'insert acétabulaire le long de l'axe d'alignement.

11. Combinaison selon la revendication 1, dans laquelle la surface d'engagement inclut un bord intérieur configuré pour être parallèle et suivre une partie d'un ligament acétabulaire transverse du patient.

12. Combinaison selon la revendication 1 associée à :
un insert de cupule acétabulaire ; et
un positionneur électronique (800, 900) attaché de manière amovible à l'arbre de l'insert acétabulaire, le positionneur électronique en mesure d'indiquer une orientation prédéterminée sur la base d'un étalonnage avec le guide acétabulaire.
